(19) **Europäisches Patentamt**
**European Patent Office**
**Office européen des brevets**

(11) **EP 4 161 411 B1**

(12) **EUROPEAN PATENT SPECIFICATION**

(45) Date of publication and mention
of the grant of the patent:
**27.11.2024 Bulletin 2024/48**

(21) Application number: **21733692.4**

(22) Date of filing: **04.06.2021**

(51) International Patent Classification (IPC):
***A61B 17/32*** (2006.01)

(52) Cooperative Patent Classification (CPC):
**A61B 17/320068;** A61B 2017/00022;
A61B 2017/00026; A61B 2017/00119;
A61B 2017/320069; A61B 2017/32007;
A61B 2217/005; A61B 2217/007

(86) International application number:
**PCT/US2021/035992**

(87) International publication number:
**WO 2021/248062 (09.12.2021 Gazette 2021/49)**

(54) **ULTRASONIC SURGICAL ASPIRATOR FOR PROBING AND ABLATING TISSUE**

CHIRURGISCHER ULTRASCHALLASPIRATOR ZUR SONDIERUNG UND ABLATION VON GEWEBE

ASPIRATEUR CHIRURGICAL À ULTRASONS POUR LE SONDAGE ET L'ABLATION DE TISSUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.06.2020 US 202063034713 P**

(43) Date of publication of application:
**12.04.2023 Bulletin 2023/15**

(73) Proprietor: **Stryker Corporation**
**Portage, MI 49002 (US)**

(72) Inventor: **DOWNEY, Adam, D.**
**Kalamazoo, MI 49009 (US)**

(74) Representative: **Röthinger, Rainer**
**Wuesthoff & Wuesthoff**
**Patentanwälte und Rechtsanwalt PartG mbB**
**Schweigerstrasse 2**
**81541 München (DE)**

(56) References cited:
**US-A1- 2012 209 303      US-A1- 2016 361 084**
**US-A1- 2018 263 652**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Background

Distinguishing between different types of patient tissue during a medical procedure can be difficult, especially when the practitioner's view of the tissue is obstructed.

US 2018/0263652 A1 discloses an ultrasonic surgical tool system for actuating a handpiece with a tip. The frequency of the drive signal applied to the handpiece drivers is a function of the equivalent of current through the mechanical components of the handpiece and tip and the frequency responsiveness of these components.

US 2012/0209303 A1 discloses a medical device for reducing the force necessary to penetrate living being tissue using a variety of reciprocating motion actuators. The reciprocating actuator drives a penetrating member, such as a needle, through the tissue at a reduced force while the device detects the passage of the penetrating member through the tissue. Upon passage of the penetrating member through the tissue, a feedback system monitors electromechanical properties of a control signal of the device and automatically modifies control based thereon, e.g., electrical power to the reciprocating actuator is automatically terminated.

US 2016/0361084 discloses a surgical instrument for cutting and coagulating tissue that includes an elongate sheath and an ultrasonic blade. An ultrasonic blade may be positioned within a sheath such that movement of the sheath relative to the blade results in the dissection and/or coagulation of tissue therebetween. Alternatively, an ultrasonic blade may be positioned within a sheath such that movement of the blade relative to the sheath results in the dissection and/or coagulation of tissue therebetween. The size and shape of distal ends of the blade and sheath determine the size and shape of the tissue being cut and/or removed from a body.

### Summary

This summary introduces a selection of concepts in a simplified form that are further described in the detailed description below. This summary is not intended to limit the scope of the claimed subject matter, and does not necessarily identify each and every key or essential feature of the claimed subject matter.

According to the invention, an ultrasonic tool system and a computer program product are provided according to the independent claims. Preferred embodiments are recited in the dependent claims.

In a first example, an ultrasonic tool system for operating an ultrasonic handpiece to probe patient tissue is provided, with the ultrasonic handpiece including a tip having a distal region for treating patient tissue and at least one driver to which the tip is coupled and to which an AC drive signal is applied to vibrate the tip. The system includes a control console configured to be coupled to the ultrasonic handpiece and to generate the AC drive signal applied to the at least one driver of the ultrasonic handpiece for vibrating the tip of the ultrasonic handpiece. The control console is further configured to: source the AC drive signal to the at least one driver of the ultrasonic handpiece, the AC drive signal including a first component at a resonant frequency of the ultrasonic handpiece and a second component at a probing frequency less than the resonant frequency; measure the voltage and current of the AC drive signal, calculate a resistance associated with the ultrasonic handpiece based on the measured voltage and the measured current; and provide at least one of an audible, visual, or tactile based on the calculated resistance.

In a first aspect, an ultrasonic tool system for operating an ultrasonic handpiece to probe patient tissue is provided, with the ultrasonic handpiece including a tip having a distal region for treating patient tissue and at least one driver to which the tip is coupled and to which an AC drive signal is applied to vibrate the tip. The system includes a control console configured to be coupled to the ultrasonic handpiece and to generate the AC drive signal applied to the at least one driver of the ultrasonic handpiece for vibrating the tip of the ultrasonic handpiece. The control console is further configured to: source the AC drive signal to the at least one driver of the ultrasonic handpiece, the AC drive signal being configured to induce vibrations at the distal region of the tip that are insufficient to ablate the patient tissue; measure the voltage and current of the AC drive signal; and provide at least one of an audible, visual, or tactile indication based on the measured voltage and current.

In a second example, an ultrasonic tool system for operating an ultrasonic handpiece to probe patient tissue is provided, with the ultrasonic handpiece including a tip having a distal region for treating patient tissue and at least one driver to which the tip is coupled and to which an AC drive signal is applied to vibrate the tip. The system comprises a control console configured to be coupled to the ultrasonic handpiece and to generate the AC drive signal applied to the at least one driver of the ultrasonic handpiece for vibrating the tip of the ultrasonic handpiece, and a switch coupled to the control console, the switch having a first setting and a second setting. Responsive to the switch being set to the first setting, the control console is configured to operate the ultrasonic handpiece in a probing mode, and responsive to the switch being set to the second setting, the control console is configured to operate the ultrasonic handpiece in an ablation mode.

In a third example, a method for probing patient tissue using an ultrasonic tool system including an ultrasonic handpiece is provided, the ultrasonic handpiece having a tip for treating patient tissue and at least one driver to which the tip is coupled and to which an AC drive signal is applied to vibrate the tip. The method comprises: sourcing the AC drive signal to the ultrasonic handpiece, the AC drive signal including a first component at a resonant frequency of the ultrasonic handpiece and a second component at a probing frequency less than the resonant frequency; measuring a voltage and current of the AC drive signal; calculating a resistance associated with the ultrasonic handpiece based on the measured voltage and the measured current; and providing at least one of an audible, visual, or tactile indication based on the calculated resistance.

In a fourth example, a method for probing patient tissue using an ultrasonic tool system including an ultrasonic handpiece is provided, the ultrasonic handpiece having a tip for treating patient tissue and at least one driver to which the tip is coupled and to which an AC drive signal is applied to vibrate the tip. The method comprises: sourcing the AC drive signal to the ultrasonic handpiece, the AC drive signal inducing vibrations at the distal region of the tip that are insufficient to ablate the patient tissue; measuring a voltage and a current of the AC drive signal; and providing at least one of an audible, visual, or tactile indication based on the measured voltage and current.

In a fifth example, a method for operating an ultrasonic tool system for probing patient tissue is provided, with the ultrasonic tool system including an ultrasonic handpiece having a tip for treating patient tissue and at least one driver to which the tip is coupled and to which an AC drive signal is applied to vibrate the tip, and a switch having a first setting and a second setting. The method comprises: sourcing the AC drive signal to the ultrasonic handpiece for vibrating the tip of the ultrasonic handpiece; monitoring a state of the switch to determine whether the switch is set to the first setting or the second setting; determining that the switch is set to the first setting; responsive to determining that the switch is set to the first setting, operating the ultrasonic handpiece in a probing mode; determining that the switch is set to the second setting; and responsive to determining that the switch is set to the second setting, operating the ultrasonic handpiece in an ablation mode.

Any of the above aspects may be combined in whole or in part.

Any of the above aspects may be utilized with any one or more of the following implementations, whether utilized individually or in combination.

Some implementations comprise the ultrasonic handpiece coupled to the control console. Some implementations comprise the ultrasonic handpiece defining a first pathway for providing suction at the distal region of the tip and a second pathway for supplying fluid to the distal region of the tip. Some implementations comprise supplying fluid to a distal region of the tip through at least a portion of the ultrasonic handpiece, and providing suction at the distal region of the tip through at least a portion of the ultrasonic handpiece.

Some implementations comprise the control console including a first sensor for measuring a voltage of the AC drive signal, a second sensor for measuring a current of the AC drive signal, and a processor coupled to the first and second sensors and configured to perform the configured functions of the of the control console. For instance, some implementations comprise the processor being configured to source the AC drive signal to the at least one driver of the ultrasonic handpiece, the AC drive signal including a first component at a resonant frequency of the ultrasonic handpiece and a second component at a probing frequency less than the resonant frequency; measure the voltage and current of the AC drive signal using the first and second sensors while the distal region of the tip is contacting the patient tissue; calculate a resistance associated with the ultrasonic handpiece based on the measured voltage and the measured current; and provide at least one of an audible, visual, or tactile indication based on the calculated resistance.

Some implementations comprise, such as by a computer program product when executed by the processor and/or control console, providing at least one of an audible, visual, or tactile indication based on the calculated resistance by identifying a property of the patient tissue based on the calculated resistance; and providing at least one of an audible, visual, or tactile indication of the identified property. Some implementations comprise the identified property being a health of the patient tissue, such as whether the patient tissue is tumorous.

Some implementations comprise the AC drive signal sourced to the ultrasonic handpiece being defined by a base signal at the resonant frequency that is amplitude modulated according to the probing frequency. Some implementations comprise the resonant frequency being approximately 25 kHz, and the probing frequency being approximately 4 Hz. The AC drive signal sourced to the ultrasonic handpiece is configured to induce vibrations of the tip that are insufficient to ablate the patient tissue. Some implementations comprise the AC drive signal sourced to the ultrasonic handpiece being configured to induce vibrations of the tip that are insufficient to ablate the patient tissue by being configured to induce vibrations at the distal region of the tip that have a peak-to-peak displacement of less than or equal to 100 micrometres.

Some implementations comprise the AC drive signal being defined as a first AC drive signal, and also comprise, such as by the computer program product when executed by the processor and/or control console, determining whether the ultrasonic tool system is set to operate in a probing mode or an ablation mode; responsive to determining that the ultrasonic tool system is set to operate in the probing mode, sourcing the first AC drive signal to the ultrasonic handpiece; and responsive to determining that the ultrasonic tool system is set to operate in the ablation mode, sourcing a second AC drive signal to the ultrasonic handpiece that is configured to induce vibrations of the tip sufficient to ablate the patient tissue. Some implementations comprise the second AC drive signal sourced to the ultrasonic handpiece being configured to induce vibrations of the tip that are sufficient to ablate the patient tissue by being configured to induce vibrations at the distal region of the tip that have a peak-to-peak displacement of greater than 100 micrometres and less than or equal to 300 micrometres.

Some implementations comprise a switch communicatively coupled to the processor and/or control console, the switch having a first setting and a second setting, and also comprise, such as by the computer program product when executed by the processor and/or control console, responsive to the switch being set to the first setting, determining that the ultrasonic tool system is set to operate in the probing mode; and responsive to the switch being set to the second setting, determining that the ultrasonic tool system is set to operate in the ablation mode.

Some implementations comprise, such as by the computer program product when executed by the processor and/or control console, responsive to determining that the ultrasonic tool system is set to operate in the ablation mode, providing the suction at the distal region of the tip through the first pathway defined by the ultrasonic handpiece; and supplying the fluid to the distal region of the tip through the second pathway defined by the ultrasonic handpiece.

Some implementations comprise, such as by the computer program product when executed by the processor and/or control console, calculating the resistance associated the ultrasonic handpiece based on the measured voltage and the measured current by calculating an equivalent of current through mechanical components of the ultrasonic handpiece based on the measured voltage and the measured current; and calculating the resistance associated with the ultrasonic handpiece based on the calculated equivalent of current through the mechanical components of the ultrasonic handpiece. Some implementations comprise, such as by the computer program product when executed by the processor and/or control console, calculating the resistance associated with the ultrasonic handpiece based on the calculated equivalent of current through the mechanical components of the ultrasonic handpiece by calculating a first amplitude of the measured voltage at the probing frequency, a second amplitude of the calculated equivalent of current through the mechanical components of the ultrasonic handpiece at the probing frequency, and a phase difference between the measured voltage and the calculated equivalent of current through the mechanical components of the ultrasonic handpiece at the probing frequency; and calculating a real part of an impedance of the ultrasonic handpiece based on the calculated first amplitude, the calculated second amplitude, and the calculated phase difference.

Some implementations comprise, such as by the computer program product when executed by the processor and/or control console, providing at least one of an audible, visual, or tactile indication based on the calculated resistance by calculating a difference between the calculated resistance and a no load resistance of the ultrasonic handpiece; and providing the at least one of the audible, visual, or tactile indication based on the calculated difference. Some implementations comprise, such as by the computer program product when executed by the processor and/or control console, providing the at least one of the audible, visual, or tactile indication based on the calculated difference by identifying a property of the patient tissue based on the calculated difference; and providing at least one of an audible, visual, or tactile indication of the identified property.

Some implementations comprise, such as in the control console, a memory storing tissue property data coupled to the processor, the tissue property data indicating potential tissue properties and, for each of the potential tissue properties, one or more values specific to the potential tissue property. Some implementations also comprise, such as by the computer program product when executed by the processor and/or control console, identifying, as a property of the patient tissue, one of the potential tissue properties indicated by the tissue property data based on the one or more values specific to the potential tissue property and the calculated resistance; and providing at least one of an audible, visual, or tactile indication of the identified property of the patient tissue.

Some implementations comprise, such as by the computer program product when executed by the processor and/or control console, identifying one of the potential tissue properties indicated by the tissue property data based on the one or more values specific to the potential tissue property and the calculated resistance by calculating a difference between the calculated resistance and a no load resistance of the ultrasonic handpiece; and identifying the one of the potential tissue properties indicated by the tissue property data based on the one or more values specific to the potential tissue property and the calculated difference.

Some implementations comprise, such as by the computer program product when executed by the processor and/or control console, determining the no load resistance of the ultrasonic handpiece by, responsive to the ultrasonic handpiece being connected to the control console: sourcing the AC drive signal to the ultrasonic handpiece while the ultrasonic handpiece is in an unloaded state; measuring a second voltage and a second current of the AC drive signal sourced to the ultrasonic handpiece, such as using the first and second sensors, while the ultrasonic handpiece is in the unloaded state; and calculating the no load resistance of the ultrasonic handpiece based on the measured second voltage and the measured second current of the AC drive signal. Some implementations comprise, such as by the computer program product when executed by the processor and/or control console, determining the no load resistance of the ultrasonic handpiece by, responsive to the ultrasonic handpiece being connected to the control console, reading data indicating the no load resistance from a memory integral with the ultrasonic handpiece.

Some implementations comprise, such as by the processor and/or controller, providing at least one of an audible, visual, or tactile indication based on the measured voltage and current by identifying a property of the patient tissue based on the measured voltage and current; and providing at least one of an audible, visual, or tactile indication of the identified property.

Some implementations comprise, such as by the computer program product when executed by the processor and/or control console, identifying a property of the patient tissue based on the measured voltage and current by calculating an equivalent of current through mechanical components of the ultrasonic handpiece based on the measured voltage and the measured current; and identifying the property of the patient tissue based on the calculated equivalent of current through the mechanical components of the ultrasonic handpiece.

Some implementations comprise the AC drive signal sourced to the ultrasonic handpiece including a first component at a resonant frequency of the ultrasonic handpiece and a second component at a probing frequency less than the resonant frequency, and also comprises, such as by the computer program product when executed by the processor and/or control console, identifying the property of the patient tissue based on the calculated equivalent of current through the mechanical components of the ultrasonic handpiece by calculating a first amplitude of the measured voltage at the probing frequency, a second amplitude of the calculated equivalent of current through the mechanical components of the ultrasonic handpiece at the probing frequency, and a phase difference between the measured voltage and the calculated equivalent of current through the mechanical components of the ultrasonic handpiece at the probing frequency; and identifying the property of the patient tissue based on the calculated first amplitude, the calculated second amplitude, and the calculated phase difference.

Some implementations comprise, such as by the computer program product when executed by the processor and/or control console, providing at least one of an audible, visual, or tactile indication based on the measured voltage and current by identifying a property of the patient tissue based on the measured voltage, the measured current, and a no load resistance of the ultrasonic handpiece; and providing at least one of an audible, visual, or tactile indication of the identified property.

Some implementations comprise, such as in the control console, a memory storing tissue property data, the tissue property data indicating potential tissue properties and, for each of the potential tissue properties, one or more values specific to the potential tissue property, and also comprise, such as by the computer program product when executed by the processor and/or control console, providing at least one of an audible, visual, or tactile indication based on the measured voltage and current by identifying, as a property of the patient tissue, one of the potential tissue properties indicated by the tissue property data based on the one or more values specific to the potential tissue property and the measured voltage and current; and providing at least one of an audible, visual, or tactile indication of the identified property of the patient tissue. Some implementations comprise, such as by the computer program product when executed by the processor and/or control console, identifying the one of the potential tissue properties indicated by the tissue property data based on the one or more values specific to the potential tissue property, the measured voltage and current, and a no load resistance of the ultrasonic handpiece.

Some implementations comprise, such as by the computer program product when executed by the processor and/or control console, determining the no load resistance of the ultrasonic handpiece by, responsive to the ultrasonic handpiece being connected to the control console: sourcing the AC drive signal to the ultrasonic handpiece while the ultrasonic handpiece is in an unloaded state; measuring a second voltage and a second current of the AC drive signal sourced to the ultrasonic handpiece, such as using the first and second sensors, while the ultrasonic handpiece is in an unloaded state; and calculating the no load resistance of the ultrasonic handpiece based on the measured second voltage and the measured second current of the AC drive signal. Some implementations comprise, such as by the computer program product when executed by the processor and/or control console, determining the no load resistance of the ultrasonic handpiece by, responsive to the ultrasonic handpiece being connected to the control console, reading data indicating the no load resistance from a memory integral with the ultrasonic handpiece.

**Brief description of the drawings**

FIG. 1 is a perspective view of an ultrasonic tool system for probing and ablating patient tissue.
FIGS. 2A and 2B are circuit diagrams representing an ultrasonic handpiece for probing and ablating patient tissue.
FIG. 3 is a schematic diagram of components of a control console for driving an ultrasonic handpiece to probe and ablate patient tissue.
FIG. 4 is a schematic diagram of components of an ultrasonic handpiece for probing and ablating patient tissue.
FIG. 5 is a flow chart of a method for operating an ultrasonic tool system to probe and ablate patient tissue.
FIG. 6 is a graph of a waveform of an AC drive signal sourced to an ultrasonic handpiece to probe patient tissue.
FIG. 7 are graphs of waveforms used to determine a property of patient tissue being probed by an ultrasonic tool system.

DETAILED DESCRIPTION

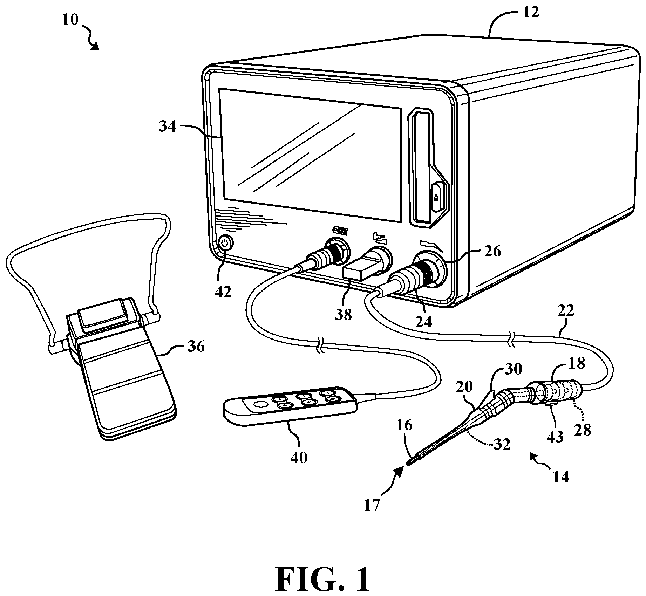

FIG. 1 illustrates an ultrasonic tool system 10 for probing and ablating patient tissue. The ultrasonic tool system 10 may include a control console 12 and an ultrasonic handpiece 14. The ultrasonic handpiece 14 may include a tip 16. During operation of the ultrasonic tool system 10, the control console 12 may source an AC drive signal to the ultrasonic handpiece 14 that causes the tip 16 to vibrate. A practitioner may then position the vibrating tip 16 against patient tissue to probe or ablate the contacted tissue.

A practitioner using the ultrasonic tool system 10 may desire to remove some types of patient tissue while leaving other types of patient tissue intact. For instance, the practitioner may desire to remove unhealthy tissue (e.g., tumorous tissue) while keeping adjacent healthy tissue intact, or to remove some kinds of patient tissue (e.g., dura mater, muscle tissue) without damaging adjacent patient tissue of a different kind (e.g., pia mater, blood vessel wall). Distinguishing between different types of patient tissue can be difficult, especially when the practitioner's view of the tissue is obstructed, or when identification of one tissue type from another tissue type is difficult to ascertain from a visual inspection. Accordingly, the ultrasonic tool system 10 may be configured to probe contacted tissue to detect and indicate the type tissue being contacted without causing tissue damage.

Specifically, the control console 12 may be configured to source an AC drive signal to the ultrasonic handpiece 14 that causes the tip 16 to vibrate in a manner insufficient to ablate contacted tissue. The control console 12 may also be configured to slowly vary the displacement amplitude of the tip 16 caused by the vibrations, and to monitor a response of the tissue as it is pushed and pulled by the vibrating tip 16. In particular, the vibrations may be longitudinal vibrations that push and pull the tissue as the displacement amplitude is varied. As the displacement amplitude of the tip 16 is increased, stiffer tissue may be harder to push and pull than relatively softer tissue. In other words, stiffer tissue may place more resistance on the ultrasonic handpiece 14 than softer tissue as the displacement amplitude of the tip 16 is increased. The control console 12 may thus be configured to track the stiffness of contacted tissue by determining a mechanical resistance of the ultrasonic handpiece 14 as a function of the varied displacement amplitude of the tip 16, and to identify a tissue property based thereon. The tissue property may indicate the type of tissue being contacted by the ultrasonic handpiece 14, such as whether the tissue is healthy or unhealthy, or the kind of tissue being contacted (e.g., blood vessel wall, dura). The control console 12 may then be configured to indicate the tissue property to the user, such as via an audible, visual, and/or tactile indication.

The above-described operation of the ultrasonic tool system 10 may occur when the ultrasonic tool system 10 is set to operate in a probing mode. Responsive to receiving the indication of the tissue property while the ultrasonic tool system 10 is operating in the probing mode, the practitioner may determine whether the tip 16 is in contact with tissue desired to be removed. If so, then the practitioner may activate an ablation mode in which the control console 12 sources an AC drive signal to the ultrasonic handpiece 14 configured to cause vibrations of the tip 16 sufficient to ablate the contacted tissue.

In addition to the tip 16, the ultrasonic handpiece 14 may include a body 18 and sleeve 20. The body 18 may define a handle for the practitioner to grasp and maneuver the ultrasonic handpiece 14. The tip 16 may be removably coupled to the body 18 so as to enable the body 18 to be used with different interchangeable tips 16. The body 18 may form a proximal end of the ultrasonic handpiece 14, and the tip 16 coupled to the body 18 may form a distal end of the ultrasonic handpiece 14. "Proximal" may be understood as towards a practitioner holding the ultrasonic handpiece 14 and away from the tissue to which the tip 16 is being applied, and "distal" may be understood as away from the practitioner and towards the tissue to which the tip 16 of the ultrasonic handpiece 14 is being applied.

The ultrasonic handpiece 14 may be removably coupled to the control console 12 through an electrical cable 22. One end the electrical cable 22 may be permanently connected to the proximal end of the body 18 of the ultrasonic handpiece 14, and the other end of the electrical cable 22 may include an adapter 24 corresponding to a socket 26 of the control console 12. The socket 26 may be shaped to receive the adapter 24, and may include electrical contacts corresponding to electrical contacts of the adapter 24 such that when the adapter 24 is fully seated in the socket 26, an electrical connection is formed between the ultrasonic handpiece 14 and control console 12.

Upon actuation of the ultrasonic handpiece 14, the control console 12 may generate and source an AC drive signal to the ultrasonic handpiece 14 over the electrical cable 22. Application of the AC drive signal to the ultrasonic handpiece 14 may cause the tip 16 of the ultrasonic handpiece 14 to vibrate. More particularly, the body 18 may define a cavity including one or more drivers 28 (three shown), such as piezoelectric drivers. Each driver 28 may be formed from a material that, upon application of an alternating electric current, undergoes a momentary expansion or contraction. The expansions and contractions of each driver 28 may be along the longitudinal axis of the driver 28, namely, the axis that extends between proximally and distally directed faces of the driver 28. The drivers 28 may be disc shaped, and may be arranged within the body 18 end to end in a stack. Insulating discs may be disposed between and tightly about adjacent drivers 28.

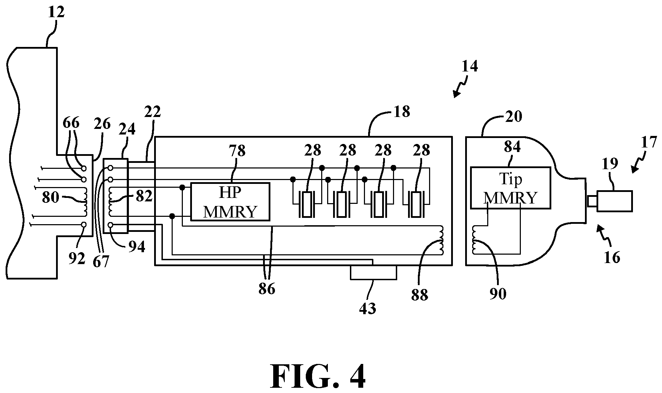

The ultrasonic handpiece 14 may be designed so that the AC drive signal received from the control console 12 is applied to each of the drivers 28, which may cause the drivers 28 to expand and contract in accordance with the AC drive signal. The drivers 28 may be coupled to the tip 16 such that the expansions and contractions of the drivers 28 induce a vibrating motion in the tip 16. Specifically, the expansions and contractions of the drivers 28 may induce back and forth vibrations along the longitudinal axis of the tip 16 that correspond to the AC drive signal sourced from the control console 12. These vibrations may cause a distal region 17 of the tip 16 to vibrate. The distal region 17 may be the portion of the ultrasonic handpiece 14 applied to patient tissue to probe and/or ablate the patient tissue. The distal region 17 may include a tip head 19 (e.g., FIG. 4), which may be formed with teeth or flutes dimensioned to remove tissue by a cutting action.

The sleeve 20 may be disposed around the tip 16, and may be formed of plastic. The proximal end of the sleeve 20 may be formed with a coupling feature for releasably coupling of the sleeve 20 to the distal end of the body 18. When disposed over the tip 16 and coupled to the body 18, the sleeve 20 may be radially spaced from the tip 16, and may be spaced longitudinally away from the distal region of the tip 16. The components of the ultrasonic handpiece 14 may thus be dimensioned so that during normal operation, the tip 16 does not contact the sleeve 20.

The ultrasonic handpiece 14 may define a pathway that extends at least partially through the ultrasonic handpiece 14 for supplying irrigating fluid to the distal region 17 of the tip 16. For example, the sleeve 20 may include a fitting 30 for receiving an irrigation line. During operation of the ultrasonic handpiece 14, irrigating fluid may be flowed through the gap between the tip 16 and the sleeve 20 via the fitting 30, and out the open distal end of the sleeve 20. The sleeve 20 may thus facilitate supplying irrigating fluid to tissue being contacted and treated by the ultrasonic handpiece 14. In an alternative example, the ultrasonic handpiece 14 may include an irrigation line that extends from the proximal end of the body 18 for receiving irrigating fluid from an irrigation source, and may define a pathway extending through the body 18 and the sleeve 20 between the irrigation line and the distal region 17 of the tip 16. During operation of the ultrasonic handpiece 14, irrigating fluid may thus be flowed through the length of the ultrasonic handpiece 14 (e.g., through the irrigation line, body 18, and sleeve 20) and out the open distal end of the sleeve 20.

The ultrasonic handpiece 14 may also define a pathway that extends at least partially through the ultrasonic handpiece 14 for providing suction at the distal region 17 of the tip 16. For instance, the ultrasonic handpiece 14 may define a lumen 32 extending from the proximal end of the body 18 through the tip 16 and to an open distal end of the tip 16.. During a procedure, suction may be provided to the lumen 32 in the proximal direction. The suction may draw the irrigating fluid applied to a surgical site and debris formed by a procedure that is entrained in the fluid. The suction may also draw tissue towards the distal region 17 of the tip 16, which may enhance the effectiveness of the tip 16 in contacting and treating tissue.

The control console 12 may include a display 34 for presenting information to a practitioner. Non-limiting examples of presented information may include an identification of the ultrasonic handpiece 14 and/or tip 16 currently connected to the control console 12, an operating state of the ultrasonic tool system 10, and a property of tissue being contacted by the tip 16 of the ultrasonic handpiece 14 as described herein. The display 34 may also be a touch screen display that enables the practitioner to provide user input to the control console 12, such as via on-screen controls. A practitioner may interact with the on-screen controls to set operational parameters for the ultrasonic tool system 10, such as a maximum tip 16 displacement level, a suction level, and an irrigation level for the ultrasonic handpiece 14.

The ultrasonic tool system 10 may also include one or more actuation devices coupled to the control console 12. Upon activation by the practitioner, each of the actuation devices may cause the control console 12 to source the AC drive signal to the ultrasonic handpiece 14 that causes the tip 16 of the ultrasonic handpiece 14 to vibrate. For instance, the one or more actuation devices may include a foot pedal 36. The foot pedal 36 may be wirelessly connected to the control console 12, such as via an adapter 38 connected to the control console 12. Upon being depressed, the foot pedal 36 may communicate an actuation signal to the control console 12 that indicates the depression. In some instances, the communicated actuation signal may vary with the extent to which the foot pedal 36 is depressed. Responsive to receiving the actuation signal, the control console 12 may source an AC drive signal to the ultrasonic handpiece 14 that causes the tip 16 to vibrate according to the current settings of the control console 12.

The ultrasonic tool system 10 may further include a remote control 40 coupled to the control console 12. Similar to the touch screen display 34, the remote control 40 may include user-selectable buttons for providing user input to the control console 12. For instance, the remote control 40 may include buttons for setting operational parameters of the ultrasonic handpiece 14, such as a maximum tip 16 displacement level, a suction level, and an irrigation level for the ultrasonic handpiece 14. The remote control 40 may also include a power button for turning on and off the control console 12. Additionally, or alternatively, the control console 12 may include an integrated power button 42 for turning on and off the control console 12.

The ultrasonic tool system 10 may also include a mode setting switch coupled to the control console 12, the switch having a probing mode setting and an ablation mode setting. A practitioner may interact with the mode setting switch to selectively set the ultrasonic tool system 10 to operate in the probing mode or the ablation mode. The control console 12 may thus be configured to monitor a state of the mode setting switch to determine whether the mode setting switch is set to the probing mode setting or the ablation mode setting. Responsive to the mode setting switch being set to the probing mode setting, the control console 12 may be configured to determine that the ultrasonic tool system 10 is set to operate in the probing mode, and to operate the ultrasonic handpiece 14 in the probing mode as described in more detail below. Conversely, responsive to the mode setting switch being set to the probing mode setting, the controller console 12 may be configured to determine that the ultrasonic tool system 10 is set to operate in the ablation mode, and to operate the ultrasonic handpiece 14 in the ablation mode as described in more detail below.

In some implementations, the mode setting switch may be a switch 43 integral with the ultrasonic handpiece 14 and coupled to the control console 12 via the electrical cable 22. Additionally or alternatively, the ultrasonic tool system 10 may include a mode setting switch integral with the foot pedal 36 and/or the remote 40. Additionally or alternatively, the control console 12 may be configured to display a virtual mode setting switch on the display 34, and a practitioner may set the mode setting switch to the ablation mode setting or the probing mode setting by interacting with the virtual mode setting switch via the touch screen interface of the display 34.

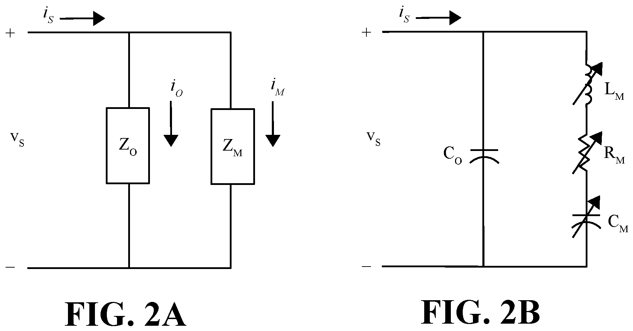

FIGS. 2A and 2B illustrate circuits representing operation of the ultrasonic handpiece 14 responsive to receiving an AC drive signal from the control console 12. The current $i_S$ of the AC drive signal sourced to the ultrasonic handpiece 14 may be broken down into two components: a current $i_O$ applied to the drivers 28 of the ultrasonic handpiece 14 and an equivalent of current $i_M$ applied to the mechanical components of the ultrasonic handpiece 14 (also referred to herein as "mechanical current $i_M$"). The mechanical components of the ultrasonic handpiece 14 may include those components that vibrate to apply force on tissue, such as the drivers 28 and tip 16.

The impedance $Z_O$ provided by the drivers 28 relative to the current $i_O$ may be primarily capacitive. Accordingly, the drivers 28 may be represented by a capacitor with capacitance $C_O$. The impedance $Z_M$ provided by the mechanical components of the ultrasonic handpiece 14 may include an inductive component, a resistive component, and a capacitive component. Accordingly, the mechanical components may be represented by an inductor with inductance $L_M$, a resistor with resistance $R_M$, and a capacitor with capacitance $C_M$. The inductance $L_M$, resistance $R_M$, capacitance $C_M$ may vary with operation of the ultrasonic handpiece 14, and at least the resistance $R_M$ may vary as a function of the tissue to which the tip 16 is applied.

The vibrations of the tip 16 of the ultrasonic handpiece 14 may be proportional to the mechanical current $i_M$. For instance, the frequency of the vibrations at the distal region 17 of the tip 16 may be equal to the frequency of the mechanical current $i_M$, and when the ultrasonic handpiece 14 is operating at resonance, the peak-to-peak displacement of the distal region 17 in micrometres may be approximately double the amplitude of the mechanical current $i_M$ in milliamps. As an example, a mechanical current $i_M$ with an amplitude of one hundred fifty milliamps may induce the distal region 17 of the tip 16 to vibrate back and forth along a path of travel that is approximately 300 micrometres. The control console 12 may thus induce vibrations at the distal region 17 with a given frequency and displacement by sourcing an AC drive signal to the ultrasonic handpiece 14 that induces a mechanical current $i_M$ with the given frequency and an amplitude corresponding to the given displacement. By Ohm's law, the mechanical current $i_M$ may be determined using the following Equation:

$$i_M = i_S - j2\pi f C_o v_s \qquad (1)$$

where $i_S$ is the current of the AC drive signal sourced to the ultrasonic handpiece 14, f is the frequency of the AC drive signal, $C_O$ is the capacitance of the drivers 28, which may be considered constant for the purposes of Equation (1) and read from a memory integral with the ultrasonic handpiece 14, and $v_s$ is the voltage of the AC drive signal. An explanation for Equation (1) can be found in Applicant's U.S. Patent No. 10,016,209. Assuming the frequency f of the AC drive signal has been previously set to achieve a desired vibratory characteristic of the ultrasonic handpiece 14 (e.g., resonance), the control console 12 may thus induce desired vibrations at the distal region 17 by setting the voltage $v_s$ of the AC drive signal so that Equation (1) results in a mechanical current in corresponding to the desired vibrations.

A characteristic integral with the ultrasonic handpiece 14 is the mechanical resonant frequency of the ultrasonic handpiece 14. The mechanical resonant frequency is the frequency at which the distal region of the tip 16 undergoes vibratory motions of a peak range. In other words, at the resonant frequency the tip 16 undergoes a motion that is larger in magnitude than a motion that would occur if the drivers 28 were vibrated at a frequency less than or greater than the resonant frequency. For a tip 16 that vibrates longitudinally, the peak range may be the largest back and forth distance.

The Applicant's U.S. Patent No. 10,016,209 discloses a means for tracking the resonant frequency of the ultrasonic handpiece 14, which may vary during operation of the ultrasonic handpiece 14. In particular, the ultrasonic handpiece 14 may be operating at resonance when the real part of the ratio of the current $i_O$ applied to the drivers 28 to the mechanical current $i_M$ is substantially equal to zero. In other words, the frequency f of the AC drive signal may correspond to the resonance frequency of the ultrasonic handpiece 14 when the following Equation is true:

$$Re\left\{\frac{j2\pi f C_0 v_s}{i_s - j2\pi f C_0 v_s}\right\} \approx 0 \qquad\qquad (2)$$

where $i_S$ is the current of the AC drive signal sourced to the ultrasonic handpiece 14, $C_0$ is the capacitance of the drivers 28, which may be considered constant for the purposes of Equation (2) and read from a memory integral with the ultrasonic handpiece 14, and $v_s$ is the voltage of the AC drive signal. To induce desired vibrations at the distal region 17 of the tip 16 during operation of the ultrasonic handpiece 14, the control console 12 may thus be configured to repeatedly alternate between determining f such that Equation (2) is true and setting the voltage $v_s$ of the AC drive signal so that Equation (1) results in the mechanical current $i_M$ corresponding to the desired vibrations.

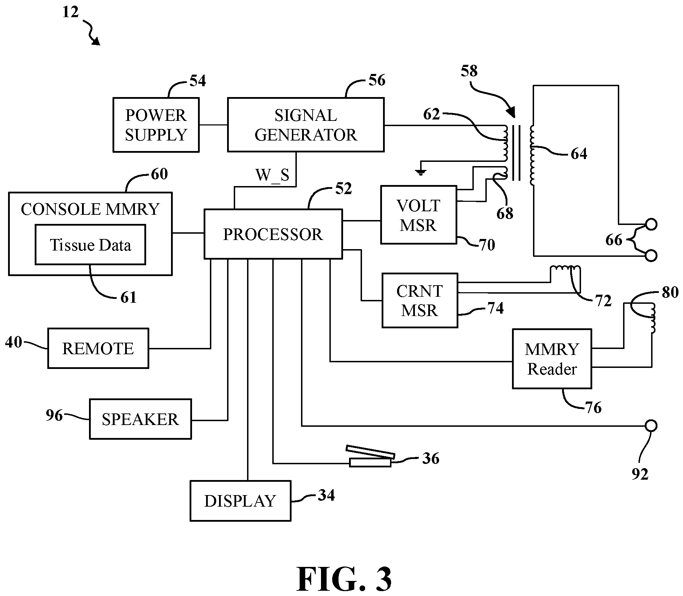

FIG. 3 illustrates components that may be present in the control console 12. The control console 12 may include a processor 52, a power supply 54, a signal generator 56, a transformer 58, and console memory 60. The processor 52 may include one or more devices selected from microprocessors, micro-controllers, digital signal processors, microcomputers, central processing units, field programmable gate arrays, programmable logic devices, state machines, logic circuits, analog circuits, digital circuits, and/or any other devices that manipulate signals (analog or digital) based on operational instructions stored in the console memory 60. The console memory 60 may include a single memory device or a plurality of memory devices including, but not limited to, read-only memory (ROM), random access memory (RAM), volatile memory, non-volatile memory, static random access memory (SRAM), dynamic random access memory (DRAM), flash memory, cache memory, and/or any other device capable of storing information. The console memory 60 may also include one or more persistent data storage devices such as a hard drive, optical drive, tape drive, non-volatile solid state device, and/or any other device capable of persistently storing information.

The processor 52 may be configured to implement the functions, features, processes, and methods of the control console 12 described herein. In particular, the processor 52 may operate under control of software embodied by computer-executable instructions residing in the console memory 60. The computer-executable instructions may be compiled or interpreted from a variety of programming languages and/or technologies, including, without limitation, and either alone or in combination, Java, C, C++, C#, Objective C, Fortran, Pascal, Java Script, Python, Perl, and PL/SQL, and may then be configured, upon execution of the processor 52, to cause the processor 52 to implement the functions, features, processes, and methods of the processor 52 described herein.

During operation of the ultrasonic tool system 10, the power supply 54 may output a constant voltage signal, typically between 1 and 250 VDC, to the signal generator 56. In some implementations, the maximum potential of the voltage output by the power supply 54 may be 150 VDC or less. The processor 52 may be configured to output a control signal (also referred to herein as a "waveform_set signal") corresponding to a desired AC drive signal to the signal generator 56. The signal generator 56, which may include an internal processor and/or amplifier, may be configured to generate an AC signal from the constant voltage signal and the waveform_set signal, such as using direct digital synthesis (DDS). More specifically, the signal generator 56 may be configured to output an AC signal having a frequency and amplitude corresponding to the waveform_set signal from the processor 52.

In some implementations, the signal generator 56 may be an amplifier, such as a Class A amplifier or the amplifier disclosed in Applicant's U.S. Patent No. 10,449,570. In this case, the processor 52 may be configured to generate a waveform_set signal with an amplitude and frequency proportional to a desired AC drive signal, such as using DDS. The amplifier may then be configured to output a signal having an amplitude and frequency based on that of the waveform_set signal.

The output of the signal generator 56 may be proportional to the desired AC drive signal indicated by the waveform_set signal, and may be applied across a primary winding 62 of the transformer 58. The AC signal from the signal generator 56 may induce the desired AC drive signal across a secondary winding 64 of the transformer 58. The secondary winding 64 may be coupled to the ultrasonic handpiece 14 through electrical contacts 66, which may be integral with the socket 26 of the control console 12, and electrical contacts 67 (FIG. 4), which may be integral with the adapter 24 coupled to the ultrasonic handpiece 14. Hence, the AC drive signal developed across the secondary winding 64 may be sourced to the ultrasonic handpiece 14 and cause the vibration of the tip 16. The processor 52 may thus be configured to source and selectively set the amplitude and frequency of the waveform of the AC drive signal applied to the drivers 28 of the ultrasonic handpiece 14, and correspondingly to control the vibrations of the distal region 17 of the tip 16 of the ultrasonic handpiece 14, via the waveform_set signal provided to the signal generator 56.

The processor 52 is configured to receive feedback data corresponding to the AC drive signal sourced to the ultrasonic handpiece 14, for example via one or more sensors of the control console 12. For example, the control console 12 may include a sensor for measuring a voltage $v_s$ of the AC drive signal sourced to the ultrasonic handpiece 14, which may include a tickler coil 68 integral with the transformer 58. The tickler coil 68 may be connected to a voltage measuring circuit 70 of the control console 12, which in turn may be connected to the processor 52. The signal across tickler coil 68 may have a known relationship to the voltage $v_s$ of the AC drive signal being sourced to the ultrasonic handpiece 14. Based on the signal across the tickler coil 68, the voltage measuring circuit 70 may generate and communicate a signal to the processor 52 representative of the potential and phase of the voltage $v_s$ of the AC drive signal being applied to the ultrasonic handpiece 14. The processor 52 may thus be configured to measure the voltage $v_s$ of the AC drive signal via the voltage measuring circuit 70 and tickler coil 68, and to make decisions based thereon.

As a further example, the control console 12 may include a sensor for measuring a current $i_s$ of the AC drive signal being sourced to the ultrasonic handpiece 14, which may include a coil 72 located in close proximity to one of the conductors that extends from the secondary winding 64 of the transformer 58 to the ultrasonic handpiece 14. The coil 72 may be connected to a current measuring circuit 74 of the control console 12, which in turn may be connected to the processor 52. The signal across the coil 72 may have a known relationship to the current $i_s$ of the AC drive signal being sourced to the ultrasonic handpiece 14. Based on the signal across coil 72, the current measuring circuit 74 may produce and communicate to the processor 52 a signal representative of the magnitude and phase of the current $i_s$ of the AC drive signal being applied to the ultrasonic handpiece 14. The processor 52 may thus be configured to measure the current $i_s$ of the AC drive signal via the current measuring circuit 74 and coil 72, and to make decisions based thereon.

In addition to software embodied by computer-executable instructions, the console memory 60 may include data supporting the functions, features, processes, and methods of the control console 12, or more particularly the processor 52, described herein. For instance, the console memory 60 may store waveform control data correlating various waveform_set signals to various AC drive signals sourced to the ultrasonic handpiece 14. The processor 52 may thus be configured to access this data to induce desired AC drive signals. As a further example, the console memory 60 may store tissue property data 61 correlating potential operating characteristics of the ultrasonic handpiece 14, such as potential modulation mechanical resistances described in more detail below, with various tissue properties. The processor 52 may thus be configured to access this data to determine a property of tissue being contacted by the ultrasonic handpiece 14 based on a determined operating characteristic of the ultrasonic handpiece 14.

The control console 12 may also include a memory reader 76 for communicating with one or more electronic memory storage devices integral with the ultrasonic handpiece 14. Referring to FIG. 4, the ultrasonic handpiece 14 may include one or more electronic memory storage devices for storing data that identifies the ultrasonic handpiece 14 and/or tip 16, and defines operational parameters specific to the ultrasonic handpiece 14 and/or tip 16. Non-limiting examples of operational parameters may include a maximum drive current for the AC drive signal, a maximum current for the mechanical current $i_M$, a maximum drive voltage for the AC drive signal, a maximum frequency for the AC drive signal, a minimum drive frequency for the AC drive signal, a capacitance $C_O$ of the drivers 28, PID coefficients, and a use history.

For instance, the body 18 of the ultrasonic handpiece 14 may include a handpiece (HP) memory 78 disposed therein. As non-limiting examples, the HP memory 78 may be an EPROM, an EEPROM, or an RFID tag. Responsive to connecting the ultrasonic handpiece 14 to the control console 12, the processor 52 may be configured to read the data stored in the HP memory 78 using the memory reader 76, and to tailor operation of the control console 12 based on the data. More particularly, the control console 12 may include a communication interface, such as a coil 80, connected to the memory reader 76. The coil 80 may be integral with the socket 26 of the control console 12. The HP memory 78 may similarly be connected to a coil 82, which may be integral with the adapter 24 of the cable 22. When the ultrasonic handpiece 14 is connected to the control console 12 via the cable 22, the coils 80, 82 may become aligned and able to inductively exchange signals. The processor 52 may then be configured to read data from and write data to the HP memory 78 over the coils 80, 82.

More particularly, the memory reader 76 may be configured to convert signals across the coil 80 into data signals readable by the processor 52. The memory reader 76 may also be configured to receive data to be written to the HP memory 78 from the processor 52, and to generate a signal across the coil 80 that causes the data to be written to the HP memory 78. The structure of the memory reader 76 may complement that of the HP memory 78. Thus, continuing with the above non-limiting examples, the memory reader 76 may be an assembly capable of reading data from and writing data to an EPROM, EEPROM, or RFID tag.

In addition or alternatively to the HP memory 78, the ultrasonic handpiece 14 may include a tip memory 84. As described above, the tip 16 may be removable from the body 18 so the body 18 can be used with varying interchangeable tips 16, and different tips 16 may have different structural characteristics and operational limitations. Accordingly, the HP memory 78 may store data identifying the body 18 and operational parameters specific to the body 18, including the capacitance $C_O$ of the drivers 28, and the tip memory 84 may store data identifying the tip 16 currently coupled to the body 18 and operational parameters specific to the tip 16. Because the tip 16 and sleeve 20 may be distributed together as a single package, the tip memory 84 may be disposed in the sleeve 20. The tip memory 84 may be the same type of memory as the HP memory 78 (e.g., an EPROM, an EEPROM, or an RFID tag).

Responsive to connecting the ultrasonic handpiece 14 to the control console 12, the processor 52 may be configured to read the data stored in the HP memory 78 and the tip memory 84 using the memory reader 76, and to tailor operation of the control console 12 to the specific body 18 and tip 16 combination coupled to the control console 12. The tip memory 84 may include values for the same operational parameters as the HP memory 78. To the extent the values for a given operational parameter differ between the HP memory 78 and the tip memory 84, the processor 52 may be configured to utilize the more restrictive value to manage operation of the ultrasonic handpiece 14. Additionally, or alternatively, to the extent the both the HP memory 78 and the tip memory 84 include a value for a given operational parameter, the processor 52 may be configured to derive a value (e.g., max drive current for the AC drive signal current) to manage operation of the ultrasonic handpiece 14 based on a combination of the values stored in memories (e.g., summing the values).

Similar to the HP memory 78, the processor 52 may read data from and write data to the tip memory 84 via the memory reader 76 and coil 80. In particular, the body 18 may include two conductors 86 extending from the proximal end to the distal end of the body 18. The proximal ends of the conductors 86 may be coupled to the coil 82, which may be integral with the adapter 24 of the cable 22. The distal ends of the conductors 86 may be coupled to another coil 88 disposed at the distal end of the body 18. A corresponding coil 90 may be disposed in a proximal end of the sleeve 20. When the sleeve 20 is disposed around the tip 16 and fitted to the body 18, the coils 88, 90 may become aligned and able to inductively exchange signals. When the body 18 is connected to the control console 12 via the cable 22, the coils 80, 82 may also become aligned and able to inductively exchange signals. The processor 52 may then read data from and write data to the tip memory 84 over the conductors 86 via inductive communication provided by the coils 80, 82 and the coils 88, 90.

The one or more electronic memory storage devices of the ultrasonic handpiece 14 may also store data for determining a property of tissue being contacted by the ultrasonic handpiece 14 when the ultrasonic tool system 10 is operating in the probing mode. As previously described, the processor 52 may be configured to determine a property of contacted tissue based on a determined operating characteristic associated with the ultrasonic handpiece 14, such as a modulation mechanical resistance described in more detail below, when the ultrasonic handpiece 14 is contacting tissue. However, a given body 18 may be used with different interchangeable tips 16, and different combinations of a body 18 and tip 16 may exhibit different baseline operating characteristics. In other words, different ultrasonic handpieces 14 may exhibit different operating characteristics when operating in an unloaded state, that is, vibrating in air and not contacting any tissue. As an example, the operating characteristics of one ultrasonic handpiece 14 when operating in an unloaded state may be about three hundred ohms, and the no load modulation mechanical resistances of other ultrasonic handpieces 14 may range between six hundred and eight hundred ohms, inclusive. The significance of a determined operating characteristic of an ultrasonic handpiece 14, such as the modulation mechanical resistance of the ultrasonic handpiece 14, relative to the property of contacted tissue may thus differ depending on the specific ultrasonic handpiece 14 being used to contact the tissue.

Accordingly, the one or more electronic memory storage devices of the ultrasonic handpiece 14 may store normalization data specific to the ultrasonic handpiece 14 for normalizing an operating characteristic of the ultrasonic handpiece 14 determined when the ultrasonic handpiece 14 is contacting patient tissue, such as data indicating a no load modulation mechanical resistance specific to the ultrasonic handpiece 14. For instance, the HP memory 78 may store a no load modulation mechanical resistance specific to the body 18, and/or the tip memory 84 may store a no load modulation mechanical resistance specific to the tip 16. As described in more detail below, this data may be determined through testing of the components of the ultrasonic handpiece 14 during production. When the ultrasonic tool system 10 is operating to probe patient tissue, the processor 52 may be configured to read this data from HP memory 78 and/or tip memory 84, and to normalize an operating characteristic determined for contacted patient tissue based thereon.

The processor 52 may also be coupled and configured to drive the display 34 of the control console 12. Specifically, the processor 52 may be configured to generate information and user interface (UI) components for presentation on the display 34. Such information depicted on display 34 may include information identifying the body 18 and the tip 16, information describing the operating state of the ultrasonic tool system 10, and information identifying a property of patient tissue being contacted by the tip 16 when the control console 12 is operating in the probing mode. When the display 34 is a touch screen display, the processor 52 may also be configured to cause the display 34 to depict images of buttons and other user-selectable components, such as the virtual mode setting switch described above. By interacting with the buttons and other user-selectable components, the practitioner may set desired operating parameters for the ultrasonic tool system 10.

The processor 52 may also be coupled to the foot pedal 36, remote 40 and a mode setting switch of the ultrasonic tool system 10, such as the switch 43 integral with the ultrasonic handpiece 14, to receive user inputs from such devices and process such inputs accordingly. For example, when the ultrasonic handpiece 14 is coupled to the control console 12, the processor 52 may become communicatively coupled to the switch 43 integral with the ultrasonic handpiece 14 via one or more electrical contacts 92 integral with the socket 26 of the control console 12 and one or more electrical contacts 94 integral with the adapter 24 coupled to the ultrasonic handpiece 14. The processor 52 may then be configured to monitor the state of the switch 43 to determine whether the ultrasonic tool system 10 is set to operate in the probing mode or ablation mode.

The processor 52 may also be coupled and configured to a drive a speaker 96 of the control console 12. For instance, responsive to determining a property of patient tissue being contacted by the tip 16, the processor 52 may be configured to play a distinct sound via the speaker 96 to indicate to the tissue property to the practitioner.

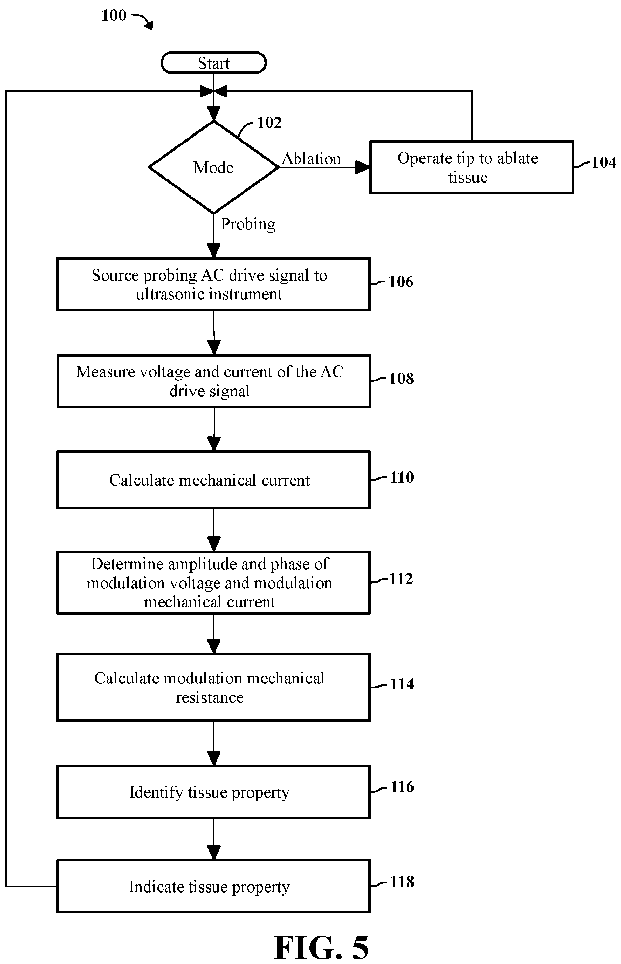

FIG. 5 illustrates a method 100 for probing patient tissue with the ultrasonic handpiece 14 to determine a property of the tissue, such as a property indicating whether the tissue is healthy or unhealthy. The method 100 may be performed by the control console 12, such as at the direction of the processor 52. More specifically, the processor 52 may be configured, such as via software stored in the console memory 60, to cause the control console 12 to perform the method 100.

In block 102, a determination may be made of whether the ultrasonic tool system 10 is set to a probing mode or an ablation mode. In the ablation mode, the control console 12, or more particularly the processor 52, may be configured to generate and source an AC drive signal to the ultrasonic handpiece 14 that causes vibrations of the tip 16 sufficient for ablating contacted tissue. In the probing mode, the control console 12, or more particularly the processor 52, may be configured to generate and source an AC drive signal to the ultrasonic handpiece 14 that causes vibrations of the tip 16 that are insufficient to ablate contacted tissue. In this latter mode, the vibrations of the tip 16 may push and pull the contacted tissue without causing damage.

The processor 52 may be configured to make the determination of block 102 based on user input selecting one of these operational modes. More particularly, the processor 52 may be configured to determine whether the ultrasonic tool system 10 is set to the probing mode or the ablation mode by monitoring the status of a mode setting switch, which may be integral with at least one of the touch screen display 34, foot pedal 36, remote control 40, or ultrasonic handpiece 14 (e.g., switch 43). Specifically, a user may interact with one of these devices to indicate one of the operational modes to the processor 52.

For example, the touch screen display 34 may illustrate an on-screen interactive element (e.g., button) for selecting between the operational modes, and the remote control 40 may likewise include an interactive control element for making the selection. The foot pedal 36 may enable user selection of one of the operational modes by the processor 52 being configured to determine whether a depression of the foot pedal 36 corresponds to the probing mode or the ablation mode. For instance, responsive to the signal received by the processor 52 from the foot pedal 36 indicating a depression less than a set threshold, the processor 52 may be configured to determine that the user desires to operate the ultrasonic tool system 10 in the probing mode. Responsive to the signal received by the processor 52 from the foot pedal 36 indicating a depression greater than or equal to the set threshold, the processor 52 may be configured to determine that the user desires to operate the ultrasonic tool system 10 in the ablation mode. Alternatively, the foot pedal 36 may have separate depressible elements for operating the ultrasonic tool system 10, one for operating the ultrasonic tool system 10 in the ablation mode, and the other for operating the ultrasonic tool system 10 in the probing mode. The switch 43 integral with the ultrasonic handpiece 14 may be configured such that depressing the switch 43 selects the probing mode and releasing the switch 43 selects the ablation mode.

Responsive to determining that the ultrasonic tool system 10 is set to operate in the ablation mode ("Ablation" branch of block 102), in block 104, the ultrasonic handpiece 14 may be operated in the ablation mode. In particular, the processor 52 may be configured to cause the control console 12 to source an AC drive signal to the ultrasonic handpiece 14 that induces vibrations of tip 16 for ablating patient tissue. The AC drive signal sourced to the ultrasonic handpiece 14 in the ablation mode may induce vibrations at the distal region of the tip 16 having vibratory cycles with a relatively high peak-to-peak displacement, such as between one hundred and three hundred micrometres, inclusive. In other words, the AC drive signal sourced to the ultrasonic handpiece 14 in the ablation mode may cause displacement of the distal region of the tip 16 back and forth along a path of travel that is between one hundred and three hundred micrometres, inclusive. Equivalently, the AC drive signal sourced to the ultrasonic handpiece 14 in the ablation mode may induce a relatively high mechanical current $i_M$, such as a mechanical current in with an amplitude between fifty and one hundred fifty milliamps, inclusive. Generation of the AC drive signal by the control console 12 in the ablation mode may be performed as described in Applicant's U.S. Patent No. 10,016,209.

In some implementations, operating the ultrasonic handpiece 14 in the ablation mode in block 104 may also include providing the suction at the distal region 17 of the tip 16 through the corresponding pathway defined by the ultrasonic handpiece 14, and supplying the fluid to the distal region 17 of the tip 16 through the corresponding pathway defined by the ultrasonic handpiece 14. In other words, responsive to determining that the ultrasonic tool system 10 is set to operate in the ablation mode, the control console 12 may be configured to initiate the supply of suction and irrigating fluid to the ultrasonic handpiece 14. Conversely, when the ultrasonic tool system 10 is set to operate in the probing mode, the control console 12 may be configured to maintain the suction and irrigation features in an inactive state.

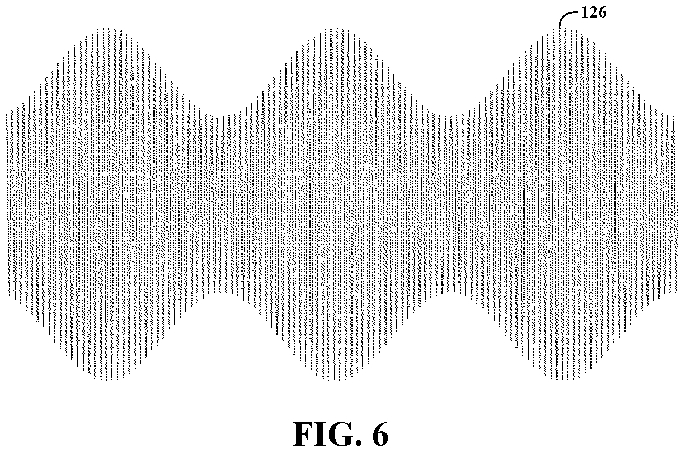

Responsive to determining that the ultrasonic tool system 10 is set to operate in the probing mode ("Probing" branch of block 102), in block 106, an AC drive signal may be sourced to the ultrasonic handpiece 14 that induces vibrations of the tip 16 for probing patient tissue, such as while the distal region of the tip 16 is contacting the patient tissue. FIG. 6 illustrates an example of an AC drive signal 126 that may be sourced to the ultrasonic handpiece 14 in the probing mode. As shown in the illustrated example, the AC drive signal 126 may include a component at a resonant frequency of the ultrasonic handpiece 14 and a component at a probing frequency. The probing frequency may be significantly less than the resonate frequency. For instance, the resonant frequency may be approximately 25 kHz (e.g., $\pm$ 1 kHz), and the probing frequency may be approximately 4 Hz (e.g., $\pm$ 1 Hz). More particularly, the AC drive signal 126 may include a base signal, such as a sinusoidal signal, at the resonant frequency with an amplitude that varies according to the probing frequency. The AC drive signal 126 sourced to the ultrasonic handpiece 14 in the probing mode may thus be an amplitude modulated signal.

The AC drive signal is configured to induce vibrations of the tip 16 that are insufficient to ablate patient tissue, and instead push and pull the patient tissue without causing damage. In particular, the AC drive signal may induce vibrations of the distal region of the tip 16 that are less in magnitude and velocity than those induced when the control console 12 is operating in the ablation mode so that the induced vibrations push and pull but do not ablate tissue. For instance, the AC drive signal sourced in the probing mode may induce vibrations at the distal region 17 of the tip 16 having vibratory cycles with a relatively low peak-to-peak displacement, such as less than or equal to 100 micrometres. In other words, while operating in probing mode, the tip 16 may vibrate back and forth along a varying path of travel that is at most 100 micrometres. Equivalently, the AC drive signal sourced to the ultrasonic handpiece 14 in the ablation mode may induce a relatively low mechanical current $i_M$, such as a mechanical current in with a varying amplitude that is less than or equal to fifty milliamps. As an example, the AC drive signal may be configured to induce a mechanical current in with an amplitude that varies between fifty milliamps and twenty-five milliamps, or varies between ten milliamps and five milliamps, according to the probing frequency.

The processor 52 may be configured to cause the control console 12 to generate the AC drive signal sourced to the ultrasonic handpiece 14 in the probing mode. In particular, the processor 52 may be configured to track the resonant frequency of the ultrasonic handpiece 14, such as by sweeping the AC drive signal between the minimum and maximum frequencies read from the electronic memory storage devices of the ultrasonic handpiece 14 and determining which frequency results in a greatest mechanical current $i_M$, or by determining a frequency f such that Equation (2) is satisfied.

Thereafter, the processor 52 may be configured to generate and communicate a waveform_set signal to the signal generator 56 that causes the signal generator 56 to develop an AC signal across the primary winding 62 of the transformer 58 proportional to the desired AC drive signal, such as using DDS. Specifically, the processor 52 may communicate a waveform-set signal that causes the signal generator 56 to produce a sinusoidal base sinusoidal waveform at the resonant frequency with an amplitude that would induce a mechanical current in with an amplitude equal to the desired maximum amplitude for the mechanical current $i_M$ (e.g., fifty milliamps, ten milliamps), to generate a sinusoidal modulation waveform at the probing frequency that varies between one and a value between zero and one (e.g., a half), and to multiply this modulation waveform by the base signal to generate the proportional AC signal across the primary winding 62.

In alternative examples, such as when the signal generator 56 is an amplifier, the processor 52 may be configured to generate a waveform-set signal that is proportional to the desired AC drive signal, such as using DDS. In particular, the processor 52 may be configured to generate a sinusoidal base signal at the tracked resonant frequency with an amplitude that would induce a mechanical current $i_M$ with an amplitude equal to the desired maximum amplitude for the mechanical current $i_M$, a sinusoidal modulation waveform at the probing frequency, and to multiply these signals to generate an amplitude modulated signal proportional to the desired AC drive signal. The processor 52 may then be configured to communicate this signal to the signal generator 56 as the waveform-set signal, which may amplify the signal to generate the desired AC drive signal across the secondary winding 64 of the transformer 58.

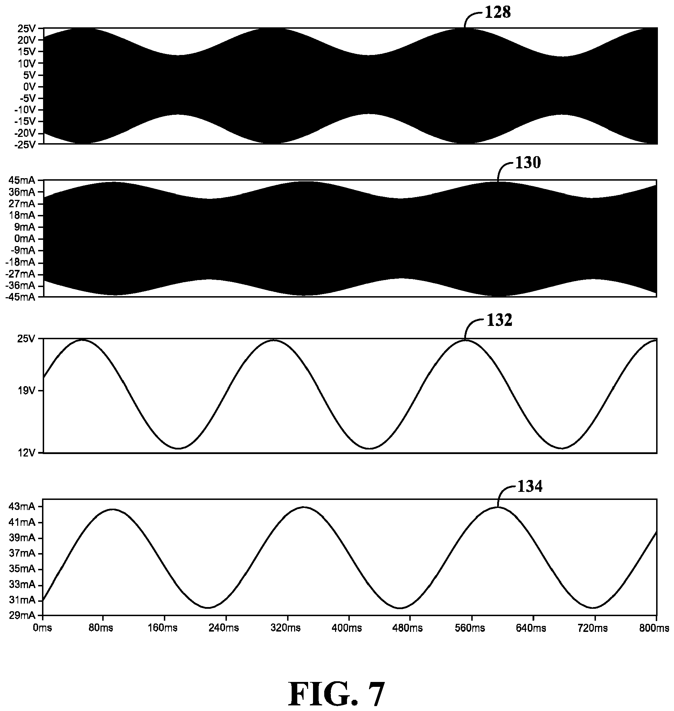

In block 108, a voltage $v_s$ and a current $i_s$ of the sourced AC drive signal may be measured while the distal region 17 of the tip 16 is contacting the patient tissue. Specifically, the processor 52 may be configured to measure the voltage $v_s$ across the ultrasonic handpiece 14 using a voltage sensor, such as the tickler coil 68 and voltage measuring circuit 70, and may be configured to measure the current $i_s$ applied to the ultrasonic handpiece 14 using a current sensor, such as the coil 72 and the current measuring circuit 74. Thereafter, in block 110, the mechanical current $i_M$ may be calculated based on the measured voltage $v_s$ and current $i_s$ of the AC drive signal. Specifically, the processor 52 may be configured to calculate the mechanical current $i_M$ by applying the measured voltage $v_s$ and current $i_s$ to Equation (1) above. FIG. 7 illustrates a voltage waveform 128 and a mechanical current waveform 130 corresponding to a measured voltage $v_s$ and a calculated mechanical current $i_M$, respectively.

During operation of the ultrasonic handpiece 14 in the probing mode, the processor 52 may be configured to continuously check that the mechanical current $i_M$ is at the resonant frequency of the ultrasonic handpiece 14 and has a magnitude equal to a target for the mechanical current $i_M$ (e.g., an amplitude varying between five and ten milliamps, varying between twenty five and fifty milliamps). For example, the processor 52 may be configured to perform cycles of determining whether the result of Equation (1) substantially equals the target for the mechanical current $i_M$ and then whether Equation (2) is substantially true. If the result of Equation (1) does not substantially equal the target for the mechanical current $i_M$, then the processor 52 may be configured to adjust the voltage $v_s$ of the AC drive signal so that the difference between the result of Equation (1) and the target for the mechanical current $i_M$ is reduced, such as by adjusting the amplitude of the waveform_set signal. Further, if Equation (2) is not substantially true, then the processor 52 may be configured to adjust the frequency of the AC drive signal so that the relationship of Equation (2) becomes substantially true, such as by adjusting the frequency of the waveform_set signal.

Referring again to the method 100, responsive to measuring the voltage $v_s$ and current $i_s$ of the sourced AC drive signal and calculating the mechanical current $i_M$, the processor 52 may be configured to determine an operating characteristic associated with the ultrasonic handpiece 14 to determine a property of the patient tissue being contacted by the ultrasonic handpiece 14. In particular, the processor 52 may be configured to calculate the resistive component of the mechanical impedance $Z_M$ of the ultrasonic handpiece 14 at the probing frequency, also referred to herein as the modulation mechanical resistance $R_{Z_{mod}}$. Specifically, as the tip 16 of the ultrasonic handpiece 14 contacts patient tissues having varied stiffness levels while the ultrasonic handpiece 14 is operating in the probing mode, the reactive components of the mechanical impedance $Z_M$ at the probing frequency may remain generally constant, while the modulation mechanical resistance $R_{Z_{mod}}$ may vary as a function of the stiffness of the tissue. Accordingly, the processor 52 may be configured to determine the modulation mechanical resistance $R_{Z_{mod}}$ to identify a property particular to the tissue being contacted by the tip 16 of the ultrasonic handpiece 14.

More particularly, in block 112, an amplitude of each of the voltage $v_s$ and mechanical current $i_M$ and a phase difference between the voltage $v_s$ and mechanical current $i_M$ at the probing frequency may be determined. The processor 52 may be configured to determine the amplitudes and phase difference by detecting an envelope of each of the voltage $v_s$ and mechanical current $i_M$, which may correspond to the probing frequency. For instance, the processor 52 may be configured to determine the upper envelopes of these signals by implementing a direct Fourier transform (DFT) algorithm that squares and low pass filters the signals or applies a Hilbert transform filter to the signals. FIG. 7 illustrates a voltage envelope waveform 132 corresponding to the upper envelope of the voltage waveform 128, and a mechanical current envelope waveform 134 corresponding to the upper envelope of the mechanical current waveform 130. As shown in the illustrated example, each of the voltage envelope waveform 132 and mechanical current envelope waveform 134 may be a sinusoidal wave at the probing frequency.

Thereafter, the processor 52 may be configured to determine the amplitudes and phase difference at the probing frequency by determining the amplitude of each envelope and the phase difference between the envelopes. The processor 52 may be configured to determine the amplitude of each envelope by executing a peak finding algorithm that determines a maximum and minimum value of the envelope, subtracting the minimum value from the maximum value, and dividing the result of the subtraction by two. The processor 52 may be configured to determine the phase difference between the envelopes by subtracting a time index corresponding to a maximum value in one envelope from the next greater time index that corresponds a maximum value in the other envelope, and dividing the result of the subtraction by the period of the envelopes (e.g., the reciprocal of the probing frequency).

In block 114, the modulation mechanical resistance $R_{Z_{mod}}$ associated with the ultrasonic handpiece 14 may be calculated based on the amplitude of each of the voltage $v_s$ and mechanical current $i_M$ at the probing frequency and the phase difference. In particular, the modulation mechanical resistance $R_{Z_{mod}}$ of the ultrasonic handpiece 14 may be equal to the real part of the mechanical impedance $Z_M$ of the ultrasonic handpiece 14 at the probing frequency. Accordingly, the processor 52 may be configured to calculate the modulation mechanical resistance $R_{Z_{mod}}$ by dividing the amplitude of the mechanical current envelope into the amplitude of the voltage envelope and multiplying the result by the cosine of the determined phase difference.

In block 116, a property of the contacted tissue may be identified based on the modulation mechanical resistance $R_{Z_{mod}}$. The identified property may indicate a type of tissue being contacted. For instance, the identified property may indicate whether the contacted tissue is healthy soft tissue or unhealthy stiff tissue, or may indicate a kind of tissue being contacted (e.g., dura, pia, blood vessel wall).

The processor 52 may be configured to identify the property of the contacted tissue by applying the modulation mechanical resistance $R_{Z_{mod}}$ to the tissue property data 61 stored in the console memory 60. The tissue property data 61 may indicate varying potential tissue properties (e.g., healthy, unhealthy, dura, pia) and, for each of the potential tissue properties, one or more values (e.g., potential modulation mechanical resistances) specific to the potential tissue property. For instance, the tissue property data 61 may define a look-up table that associates various potential modulation mechanical resistances $R_{Z_{mod}}$ with varying tissue health ratings. The greater the potential modulation mechanical resistance $R_{Z_{mod}}$, the poorer tissue health rating that may be associated with the potential modulation mechanical resistance $R_{Z_{mod}}$. As a further example, the tissue property data 61 may define a threshold such that potential modulation mechanical resistances $R_{Z_{mod}}$ less than the threshold are associated with healthy tissue or one kind of tissue and potential modulation mechanical resistances $R_{Z_{mod}}$ greater than the threshold are associated with unhealthy tissue or another kind of tissue.

In some instances, the practitioner may be able to define the tissue property data 61 used to determine tissue properties in the probing mode. For example, the practitioner may interact with the control console 12 via the touch screen display 34 to specify a tissue type desired to be removed or detected and/or a tissue type desired to be left intact. The console memory 60 may include tissue property data 61 for each possible tissue type available for user selection, and the processor 52 may be configured to retrieve and use the tissue property data 61 corresponding to the practitioner's selections to determine whether the tissue being contacted by the ultrasonic handpiece 14 has a property corresponding to a tissue type selected for removal or a tissue type selected to be left intact. The practitioner may also be able to define the thresholds and/or lookup tables directly.

In some instances, determining a tissue property based on the calculated modulation mechanical resistance

$R_{Z_{mod}}$ may include normalizing the modulation mechanical resistance $R_{Z_{mod}}$ based on the specific ultrasonic handpiece 14 coupled to the control console 12 and contacting the tissue, and applying the normalized modulation mechanical resistance to the tissue property data 61 as described above. As previously described, a given body 18 may be configured to be utilized with a variety of interchangeable tips 16, and different combinations of a body 18 and tip 16 may exhibit different mechanical resistances at the probing frequency when operating in an unloaded condition, that is, vibrating while not contacting any tissue, also referred to herein as a no load modulation mechanical resistance. Correspondingly, different combinations of a body 18 and tip 16 may exhibit a different modulation mechanical resistance $R_{Z_{mod}}$ when contacting the same tissue. The significance of a given modulation mechanical resistance $R_{Z_{mod}}$ relative to a property of contacted tissue may therefore differ depending on the specific ultrasonic handpiece 14, or more particularly the specific body 18 and/or tip 16, being used to contact the tissue.

The processor 52 may thus be configured to identify a property of the contacted tissue based on the calculated modulation mechanical resistance $R_{Z_{mod}}$ by identifying a no load modulation mechanical resistance specific to the ultrasonic handpiece 14 being used to contact the tissue, subtracting the no load modulation mechanical resistance from the calculated modulation mechanical resistance $R_{Z_{mod}}$, and applying this normalized modulation mechanical resistance to the tissue property data 61 to determine a corresponding tissue property as described above. The processor 52 may be configured to determine the no load mechanical resistance of the ultrasonic handpiece 14 by running a test of the ultrasonic handpiece 14 when the ultrasonic handpiece 14 is initially connected to the control console 12 and operating in an unloaded state (e.g., vibrating while not contacting any patient tissue). In particular, responsive to the ultrasonic handpiece 14 being connected to the control console 12 and to the control console 12 being powered on, before the ultrasonic handpiece 14 is contacting any tissue, the processor 52 may be configured to implement blocks 106 to 114 of the method 100 to calculate a modulation mechanical resistance that is assumed to correspond to the no load condition.

Alternatively, the no load modulation mechanical resistance specific to the ultrasonic handpiece 14 may be determined from data previously stored in and read from one or more electronic memory storage devices integral with the ultrasonic handpiece 14, such as the HP memory 78 and/or the tip memory 84. The variation of no load modulation mechanical resistances among different ultrasonic handpieces 14 may be primarily due to the utilization of different tips 16 in the ultrasonic handpieces 14. Accordingly, data for identifying a no load modulation mechanical resistance for an ultrasonic handpiece 14 may be stored in the tip memory 84 distributed with the tip 16 of the ultrasonic handpiece 14. For instance, during production of a tip 16, a no load modulation mechanical resistance for the tip 16 may be determined by coupling the tip 16 to a body 18 to form an ultrasonic handpiece 14, coupling this ultrasonic handpiece 14 to a control console 12, and causing this control console 12 to implement blocks 106 to 114 when the tip 16 is not contacting any tissue to determine a no load modulation mechanical resistance for the tip 16. This no load modulation resistance may then be stored in the tip memory 84 distributed with the tip 16. Thereafter, responsive to an ultrasonic handpiece 14 with the tip 16 being connected to the control console 12 and to the control console 12 being powered on in preparation for an operation, the processor 52 may be configured to read the no load mechanical resistance from the tip memory 84, and to use this value as the normalizing no load modulation mechanical resistance for the ultrasonic handpiece 14.

Alternatively, both the HP memory 78 and tip memory 84 of the ultrasonic handpiece 14 may store data for determining the no load modulation mechanical resistance for the ultrasonic handpiece 14. Specifically, the HP memory 78 of the body 18 may store data indicating a no load modulation mechanical resistance for the body 18, and the tip memory 84 distributed with the tip 16 may store data indicating a no load modulation mechanical resistance for the tip 16. The no load modulation mechanical resistance for the body 18 may be determined during production by coupling the body 18 to a control console 12 without a tip 16 and causing the control console 12 to implement blocks 106 to 114 without contacting any tissue with the body 18. The no load modulation mechanical resistance for the tip 16 may be determined during production by coupling the tip 16 to a body 18 to form an ultrasonic handpiece 14, coupling this ultrasonic handpiece 14 to a control console 12, causing the control console 12 to implement blocks 106 to 114 without the ultrasonic handpiece 14 contacting any tissue to determine a no load modulation mechanical resistance of the ultrasonic handpiece 14, and subtracting a previously determined no load modulation mechanical resistance for the body 18 from this no load modulation mechanical resistance to determine the no load modulation mechanical resistance for the tip 16. Thereafter, responsive to an ultrasonic handpiece 14 with the body 18 and tip 16 being connected to the control console 12 and to the control console 12 being powered on in preparation for an operation, the processor 52 may be configured to determine the no load modulation mechanical resistance for the ultrasonic handpiece 14 by reading the no load mechanical resistance specific to the body 18 from the HP memory 78, reading the no load mechanical resistance specific to the tip 16 from the tip memory 84, and determining a no load mechanical resistance specific to the ultrasonic handpiece 14 based on the read data (e.g., summing the read no load modulated mechanical resistances).

Automatically running a test of an ultrasonic handpiece 14 upon its connection to the control console 12 for an operation or storing data indicating the no load resistance specific to the ultrasonic handpiece 14 in the HP memory 78 and/or tip memory 84 integral with the ultrasonic handpiece 14 enables the control console 12 to accurately probe patient tissue with different ultrasonic handpieces 14, or more particularly different combinations of a body 18 and tip 16, by normalizing the calculated modulation mechanical resistance $R_{Z_{mod}}$ of the ultrasonic handpiece 14 to the specific ultrasonic handpiece 14 being used. During the time in which the processor 52 is determining the no load modulation mechanical resistance for a connected ultrasonic handpiece 14, such as by running a test of the ultrasonic handpiece 14 or reading previously stored data from the ultrasonic handpiece 14, the processor 52 may be configured to cause the display 34 to show a notification indicating that the ultrasonic tool system 10 is being initialized and to not place the tip 16 against any tissue, and may be configured to disable user input from causing the ultrasonic handpiece 14 to operate. Responsive to determining the no load modulation mechanical resistance for the ultrasonic handpiece 14, the processor 52 may be configured to cause the display 34 to indicate that the ultrasonic tool system 10 is ready for use, and to enable user input to cause the ultrasonic handpiece 14 to operate.

Referring again to FIG. 5, in block 118, the tissue property may be indicated to the practitioner. In particular, the processor 52 may be configured to provide a visual indicator corresponding to the identified tissue property, such as via the display 34 or a visual indicator integral with the ultrasonic handpiece 14, an audible indicator corresponding to the identified tissue property via the speaker 96, and/or a tactile indicator corresponding to the identified tissue property via a vibration of the ultrasonic handpiece 14. To provide the tactile indication, the processor 52 may be configured to source an AC drive signal to the ultrasonic handpiece 14 that causes a distinct vibration pattern that is insufficient to ablate tissue and may be felt by the practitioner gripping the ultrasonic handpiece 14. For example, the processor 52 may be configured to source an AC drive signal to the ultrasonic handpiece 14 that includes on pulses separated by off periods, which may induce spaced apart ultrasonic vibrations of the tip 16 insufficient to ablate tissue.

If the identified property indicates a tissue type corresponding to a binary tissue condition (e.g., the property indicates whether or not the contacted tissue is to be ablated, the property indicates whether or not the contacted tissue is healthy tissue, the property indicates whether or not the contacted tissue is a kind of tissue set by the practitioner), then the processor 52 may be configured to provide an indication of the identified property if the property corresponds to one of the states of the binary condition, and provide no indication if the property corresponds to the other state of the binary condition. For instance, if the identified property indicates that the contacted tissue is healthy, the processor 52 may be configured to provide no indication of the property, and if the identified property indicates that the contacted tissue is unhealthy, then the processor 52 may be configured to provide a visual, audible, and/or tactile indication of the property as described above. Alternatively, the processor 52 may be configured to provide an indication of the property regardless of the state represented by the property.

If the identified property indicates a tissue type corresponding to a tissue condition of varying severity (e.g., varying levels of unhealthy tissue), then the processor 52 may be configured to provide an indication of the identified property that is varied in accordance with the severity indicated by the property. For instance, if one identified property indicates that contacted tissue has a poor health rating, then the processor 52 may be configured to provide one audible, visual, and/or tactile indication, and if another identified property indicates that contacted tissue has a poorer health rating, then the processor 52 may be configured to provide a different audible, visual, and/or tactile indication indicative of the poorer health rating relative to the former health rating. As some non-limiting examples, the processor 52 may be configured to indicate the poorer health rating by displaying an indication having a larger magnitude on the display 34 than that for the former health rating, sounding the speaker 96 at a louder volume or with beeps at a greater frequency than that for the former health rating, and/or vibrating the ultrasonic handpiece 14 such that the on pulses occur at a greater frequency than that for the former health rating.

An ultrasonic tool system and method for probing and/or ablating patient tissue are described herein. Distinguishing between different types of patient tissue during a medical procedure can be difficult, especially when the practitioner's view of the tissue is obstructed, or when identification of one tissue type from another tissue type is difficult to ascertain from a visual inspection. Accordingly, examples are described herein that provide for identifying a property of patient tissue using an ultrasonic handpiece without damaging the tissue. The identified tissue property may be indicated to the practitioner, who may then decide whether to ablate the tissue using the ultrasonic tool system or to leave the tissue intact based on the indication.

The computer-executable program code described herein is capable of being individually or collectively distributed as a program product in a variety of different forms. In particular, the program code may be distributed using a computer-readable storage medium having computer-readable program instructions thereon for causing a processor to carry out any of the steps executable by a processor as described herein.

Computer-readable storage media, which is inherently non-transitory, may include volatile and non-volatile, and removable and non-removable tangible media implemented in any method or technology for storage of information, such as computer-readable instructions, data structures, program modules, or other data. Computer-readable storage media may further include RAM, ROM, erasable programmable read-only memory (EPROM), electrically erasable programmable read-only memory (EEPROM), flash memory or other solid state memory technology, portable compact disc read-only memory (CD-ROM), or other optical storage, magnetic cassettes, magnetic tape, magnetic disk storage or other magnetic storage devices, or any other medium that can be used to store the desired information and which can be read by a computer. A computer-readable storage medium should not be construed as transitory signals per se (e.g., radio waves or other propagating electromagnetic waves, electromagnetic waves propagating through a transmission media such as a waveguide, or electrical signals transmitted through a wire). Computer-readable program instructions may be downloaded to a computer, another type of programmable data processing apparatus, or another device from a computer-readable storage medium or to an external computer or external storage device via a network.

Computer-readable program instructions stored in a computer-readable medium may be used to direct a computer, other types of programmable data processing apparatus, or other devices to function in a particular manner, such that the instructions stored in the computer-readable medium produce an article of manufacture including instructions that implement the functions, acts, and/or operations specified in the flow-charts, sequence diagrams, and/or block diagrams. The computer program instructions may be provided to one or more processors of a general purpose computer, a special purpose computer, or other programmable data processing apparatus to produce a machine, such that the instructions, which execute via the one or more processors, cause a series of computations to be performed to implement the functions, acts, and/or operations specified in the flow-charts, sequence diagrams, and/or block diagrams.

In certain alternative examples, the functions, acts, and/or operations specified in the flow-charts, sequence diagrams, and/or block diagrams may be reordered, processed serially, and/or processed concurrently consistent with computer program product embodiments of the invention. Moreover, any of the flow-charts, sequence diagrams, and/or block diagrams may include more or fewer blocks than those illustrated consistent with computer program product embodiments of the invention.

While the invention has been illustrated by a description of various examples and while these examples have been described in considerable detail, it is not the intention of the Applicant to restrict or in any way limit the scope of the appended claims to such detail. Additional advantages and modifications will readily appear to those skilled in the art. The invention in its broader aspects is therefore not limited to the specific details, representative apparatus and computer program product, and illustrative examples shown and described. Accordingly, departures may be made from such details without departing from the scope of the disclosure. The invention is defined by the appended claims.

## Claims

1. An ultrasonic tool system (10) for probing patient tissue, the system (10) comprising:

    an ultrasonic handpiece (14) comprising a tip (16) having a distal region (17) for treating patient tissue and at least one driver (28) to which the tip (16) is coupled and to which an AC drive signal is applied to vibrate the tip (16), the ultrasonic handpiece (14) defining a first pathway for providing suction at the distal region (17) of the tip (16) and a second pathway for supplying fluid to the distal region (17) of the tip (16); and
    a control console (12) coupled to the ultrasonic handpiece (14) and configured to generate the AC drive signal applied to the at least one driver (28) of the ultrasonic handpiece (14) for vibrating the tip (16) of the ultrasonic handpiece (14), the control console (12) comprising:

        a first sensor for measuring a voltage of the AC drive signal;
        a second sensor for measuring a current of the AC drive signal; and
        a processor (52) coupled to the first and second sensors and configured to:

            source (106) the AC drive signal to the at least one driver (28) of the ultrasonic handpiece (14), the AC drive signal inducing vibrations at the distal region (17) of the tip (16) that are insufficient to ablate the patient tissue;
            measure (108) the voltage and current of the AC drive signal using the first and second sensors; and
            provide at least one of an audible, visual, or tactile indication based on the measured voltage and current.

2. The system (10) of claim 1, wherein the processor (52) is configured to provide at least one of an audible, visual, or tactile indication based on the measured voltage and current by being configured to:

    identify a property of the patient tissue based on the measured voltage and current; and
    provide at least one of an audible, visual, or tactile indication of the identified property.

3. The system (10) of claim 2, wherein the processor (52) is configured to identify a property of the patient tissue based on the measured voltage and current by being configured to:

    calculate an equivalent of current through mechanical components of the ultrasonic handpiece (14) based on the measured voltage and the measured current; and
    identify the property of the patient tissue based on the calculated equivalent of current through the mechanical components of the ultrasonic handpiece (14).

4. The system (10) of claim 3, wherein the AC drive signal sourced to the ultrasonic handpiece (14) includes a first component at a resonant frequency of the ultrasonic handpiece (14) and a second component at a probing frequency less than the resonant frequency, and the processor (52) is configured to identify the property of the patient tissue based on the calculated equivalent of current through the mechanical components of the ultrasonic handpiece (14) by being configured to:

   calculate a first amplitude of the measured voltage at the probing frequency, a second amplitude of the calculated equivalent of current through the mechanical components of the ultrasonic handpiece (14) at the probing frequency, and a phase difference between the measured voltage and the calculated equivalent of current through the mechanical components of the ultrasonic handpiece (14) at the probing frequency; and
   identify the property of the patient tissue based on the calculated first amplitude, the calculated second amplitude, and the calculated phase difference.

5. The system (10) of any one of claims 1-4, wherein the processor (52) is configured to provide at least one of an audible, visual, or tactile indication based on the measured voltage and current by being configured to:

   identify a property of the patient tissue based on the measured voltage, the measured current, and a no load resistance of the ultrasonic handpiece (14); and
   provide at least one of an audible, visual, or tactile indication of the identified property.

6. The system (10) of any one of claims 1-5, wherein the control console (12) further comprises a memory (60) storing tissue property data (61) coupled to the processor (52), the tissue property data (61) indicating potential tissue properties and, for each of the potential tissue properties, one or more values specific to the potential tissue property, and the processor (52) is configured to provide at least one of an audible, visual, or tactile indication based on the measured voltage and current by being configured to:

   identify, as a property of the patient tissue, one of the potential tissue properties indicated by the tissue property data (61) based on the one or more values specific to the potential tissue property and the measured voltage and current; and
   provide at least one of an audible, visual, or tactile indication of the identified property of the patient tissue.

7. The system (10) of claim 6, wherein the processor (52) is configured to identify the one of the potential tissue properties indicated by the tissue property data (61) based on the one or more values specific to the potential tissue property, the measured voltage and current, and a no load resistance of the ultrasonic handpiece (14).

8. The system (10) of claims 5 or 7, wherein the processor (52) is configured to determine the no load resistance of the ultrasonic handpiece (14) by being configured to, responsive to the ultrasonic handpiece (14) being connected to the control console (12):

   i) source the AC drive signal to the ultrasonic handpiece (14) while the ultrasonic handpiece (14) is in an unloaded state;

      measure a second voltage and a second current of the AC drive signal sourced to the ultrasonic handpiece (14) using the first and second sensors while the ultrasonic handpiece (14) is in the unloaded state; and
      calculate the no load resistance of the ultrasonic handpiece (14) based on the measured second voltage and the measured second current of the AC drive signal; or

   ii) read data indicating the no load resistance from a memory (78, 84) integral with the ultrasonic handpiece (14).

9. A computer program product comprising instructions which, when the program is executed by the processor (52) of the system of claim 1; cause the processor (52) of the system of claim 1 to carry out steps of:

   i) generating and sourcing (106) an AC drive signal to an ultrasonic handpiece (14) of an ultrasonic tool system (10), the AC drive signal being configured to cause vibrations of a tip (16) of the ultrasonic handpiece (14) that are insufficient to ablate contacted tissue;
   ii) receiving feedback data corresponding to the AC drive signal comprising a measured voltage of the AC drive signal and a measured current of the AC drive signal; and
iii) providing at least one of an audible, visual, or tactile indication based on the measured voltage and current.

10. The computer program product of claim 9, wherein providing at least one of an audible, visual, or tactile indication based on the measured voltage and current comprises:

identifying (116) a property of patient tissue based on the measured voltage and current; and
providing (118) at least one of audible, visual, or tactile indication of the identified property.

11. The computer program product of claim 10, wherein identifying a property of the patient tissue based on the measured voltage and current comprises:

calculating an equivalent of current through mechanical components of the ultrasonic handpiece (14) based on the measured voltage and the measured current; and
identifying the property of the patient tissue based on the calculated equivalent of current through the mechanical components of the ultrasonic handpiece (14); and, optionally,
wherein the AC drive signal sourced to the ultrasonic handpiece (14) includes a first component at a resonant frequency of the ultrasonic handpiece (14) and a second component at a probing frequency less than the resonant frequency, and identifying the property of the patient tissue based on the calculated equivalent of current through the mechanical components of the ultrasonic handpiece (14) comprises:

calculating a first amplitude of the measured voltage at the probing frequency, a second amplitude of the calculated equivalent of current through the mechanical components of the ultrasonic handpiece (14) at the probing frequency, and a phase difference between the measured voltage and the calculated equivalent of current through the mechanical components of the ultrasonic handpiece (14) at the probing frequency; and
identifying the property of the patient tissue based on the calculated first amplitude, the calculated second amplitude, and the calculated phase difference.

12. The computer program product of any one of claims 9-11, further comprising instructions which, when the program is executed by the processor (52) of the system of claim 1; cause the processor (52) of the system of claim 1 to carry out a step of:
identifying the property of the patient tissue based on the measured voltage, the measured current, and a no load resistance of the ultrasonic handpiece (14).

13. The computer program product of any one of claims 9-12, wherein the ultrasonic tool system (10) further comprises a memory (60) storing tissue property data (61), the tissue property data (61) indicating potential tissue properties and, for each of the potential tissue properties, one or more values specific to the potential tissue property, and providing at least one of an audible, visual, or tactile indication based on the measured voltage and current comprises:

identifying, as a property of the patient tissue, one of the potential tissue properties indicated by the tissue property data (61) based on the one or more values specific to the potential tissue property and the measured voltage and current; and
providing at least one of an audible, visual, or tactile indication of the identified property of the patient tissue.

14. The computer program product of claim 13, further comprising instructions which, when the program is executed by the processor (52) of the system of claim 1; cause the processor (52) of the system of claim 1 to carry out a step of:
identifying the one of the potential tissue properties indicated by the tissue property data (61) based on the one or more values specific to the potential tissue property, the measured voltage and current, and a no load resistance of the ultrasonic handpiece (14).

15. The computer program product of claims 12 or 14, further comprising instructions which, when the program is executed by the processor (52) of the system of claim 1; cause the processor (52) of the system of claim 1 to carry out a step of determining the no load resistance of the ultrasonic handpiece (14) by:

i) positioning the ultrasonic handpiece (14) in an unloaded state; and
while the ultrasonic handpiece (14) is positioned in the unloaded state:

sourcing (106) the AC drive signal to the ultrasonic handpiece (14);

measuring a second voltage and a second current of the AC drive signal sourced to the ultrasonic handpiece; and

calculating the no load resistance of the ultrasonic handpiece (14) based on the measured second voltage and the measured second current of the AC drive signal; or

ii) reading data indicating the no load resistance from a memory (78, 84) integral with the ultrasonic handpiece (14).

**Patentansprüche**

1. Ultraschallwerkzeugsystem (10) zum Untersuchen von Patientengewebe, wobei das System (10) umfasst:

ein Ultraschallhandstück (14), das eine Spitze (16) mit einem distalen Bereich (17) zum Behandeln von Patientengewebe und mindestens einen Mitnehmer (28) umfasst, mit dem die Spitze (16) gekoppelt ist und an den ein Wechselstrom-Antriebssignal angelegt wird, um die Spitze (16) in Schwingung zu versetzen, wobei das Ultraschallhandstück (14) einen ersten Kanal zum Bereitstellen einer Saugfunktion an dem distalen Bereich (17) der Spitze (16) und einen zweiten Kanal zum Zuführen von Fluid zu dem distalen Bereich (17) der Spitze (16) definiert; und

eine Steuerungskonsole (12), die mit dem Ultraschallhandstück (14) gekoppelt und eingerichtet ist, um das Wechselstrom-Antriebssignal zu erzeugen, das an den mindestens einen Mitnehmer (28) des Ultraschallhandstücks (14) angelegt wird, um die Spitze (16) des Ultraschallhandstücks (14) in Schwingung zu versetzen, wobei die Steuerungskonsole (12) umfasst:

einen ersten Sensor zum Messen einer Spannung des Wechselstrom-Antriebssignals; einen zweiten Sensor zum Messen eines Stroms des Wechselstrom-Antriebssignals; und einen Prozessor (52), der mit dem ersten und dem zweiten Sensor gekoppelt und eingerichtet ist, um:

das Wechselstrom-Antriebssignal dem mindestens einen Mitnehmer (28) des Ultraschall-Handstücks (14) zuzuführen (106), wobei das Wechselstrom-Antriebssignal in dem distalen Bereich (17) der Spitze (16) Schwingungen verursacht, die nicht ausreichend sind, um das Patientengewebe abzutragen; die Spannung und den Strom des Wechselstrom-Antriebssignals unter Verwendung des ersten und des zweiten Sensors zu messen (108); und einen akustischen, einen visuellen und/oder einen taktilen Hinweis auf der Grundlage der gemessenen Spannung und des gemessenen Stroms bereitzustellen.

2. System (10) nach Anspruch 1, wobei der Prozessor (52) eingerichtet ist, um auf der Grundlage der gemessenen Spannung und des gemessenen Stroms einen akustischen, einen visuellen und/oder einen taktilen Hinweis bereitzustellen, indem er eingerichtet ist, um:

eine Eigenschaft des Patientengewebes auf der Grundlage der gemessenen Spannung und des gemessenen Stroms zu identifizieren; und einen akustischen, einen visuellen und/oder einen taktilen Hinweis der identifizierten Eigenschaft bereitzustellen.

3. System (10) nach Anspruch 2, wobei der Prozessor (52) eingerichtet ist, um eine Eigenschaft des Patientengewebes auf der Grundlage der gemessenen Spannung und des gemessenen Stroms zu identifizieren, indem er eingerichtet ist, um:

ein Äquivalent des Stroms durch mechanische Komponenten des Ultraschallhandstücks (14) auf der Grundlage der gemessenen Spannung und des gemessenen Stroms zu berechnen; und die Eigenschaft des Patientengewebes auf der Grundlage des berechneten Äquivalents des Stroms durch die mechanischen Komponenten des Ultraschallhandstücks (14) zu identifizieren.

4. System (10) nach Anspruch 3, wobei das Wechselstrom-Antriebssignal, das dem Ultraschall-Handstück (14) zugeführt wird, eine erste Komponente bei einer Resonanzfrequenz des Ultraschall-Handstücks (14) und eine zweite Komponente bei einer Untersuchungsfrequenz, die niedriger als die Resonanzfrequenz ist, enthält, und der Prozessor (52) eingerichtet ist, um die Eigenschaft des Patientengewebes auf der Grundlage des berechneten Äquivalents des Stroms durch die mechanischen Komponenten des Ultraschall-Handstücks (14) zu identifizieren, indem er eingerichtet ist, um:

eine erste Amplitude der gemessenen Spannung bei der Untersuchungsfrequenz, eine zweite Amplitude des berechneten Äquivalents des Stroms durch die mechanischen Komponenten des Ultraschallhandstücks (14) bei der Untersuchungsfrequenz und eine Phasendifferenz zwischen der gemessenen Spannung und dem berechneten Äquivalent des Stroms durch die mechanischen Komponenten des Ultraschallhandstücks (14) bei der Untersuchungsfrequenz zu berechnen; und

die Eigenschaft des Patientengewebes auf der Grundlage der berechneten ersten Amplitude, der berechneten zweiten Amplitude und der berechneten Phasendifferenz zu identifizieren.

5. System (10) nach einem der Ansprüche 1-4, wobei der Prozessor (52) eingerichtet ist, um auf der Grundlage der gemessenen Spannung und des gemessenen Stroms einen akustischen, einen visuellen und/oder einen taktilen Hinweis bereitzustellen, indem er eingerichtet ist, um:

eine Eigenschaft des Patientengewebes auf der Grundlage der gemessenen Spannung, des gemessenen Stroms und eines Nulllast-Widerstands des Ultraschall-Handstücks (14) zu identifizieren; und

einen akustischen, einen visuellen und/oder einen taktilen Hinweis der identifizierten Eigenschaft bereitzustellen.

6. System (10) nach einem der Ansprüche 1 bis 5, wobei die Steuerungskonsole (12) ferner einen Speicher (60) umfasst, der Gewebeeigenschaftsdaten (61) speichert und der mit dem Prozessor (52) gekoppelt ist, wobei die Gewebeeigenschaftsdaten (61) potenzielle Gewebeeigenschaften und für jede der potenziellen Gewebeeigenschaften einen oder mehrere Werte, die für die potenzielle Gewebeeigenschaft spezifisch sind, angeben, und der Prozessor (52) eingerichtet ist, um auf der Grundlage der gemessenen Spannung und des gemessenen Stroms einen akustischen, einen visuellen und/oder einen taktilen Hinweis bereitzustellen, indem er eingerichtet ist, um:

eine der potenziellen Gewebeeigenschaften, die durch die Gewebeeigenschaftsdaten (61) angegeben werden, als eine Eigenschaft des Patientengewebes auf der Grundlage des einen oder der mehreren Werte, die für die potenzielle Gewebeeigenschaft spezifisch sind, und der gemessenen Spannung und des gemessenen Stroms zu identifizieren; und

einen akustischen, einen visuellen und/oder einen taktilen Hinweis der identifizierten Eigenschaft des Patientengewebes bereitzustellen.

7. System (10) nach Anspruch 6, wobei der Prozessor (52) eingerichtet ist, um die eine der potenziellen Gewebeeigenschaften, die durch die Gewebeeigenschaftsdaten (61) angegeben werden, auf der Grundlage des einen oder der mehreren Werte, die für die potenzielle Gewebeeigenschaft spezifisch sind, der gemessenen Spannung und des gemessenen Stroms und eines Nulllast-Widerstands des Ultraschallhandstücks (14) zu identifizieren.

8. System (10) nach Anspruch 5 oder 7, wobei der Prozessor (52) eingerichtet ist, um den Nulllast-Widerstand des Ultraschall-Handstücks (14) zu bestimmen, indem er eingerichtet ist, um als Reaktion darauf, dass das Ultraschall-Handstück (14) mit der Steuerungskonsole (12) verbunden ist,:

i) das Wechselstrom-Antriebssignal dem Ultraschall-Handstück (14) zuzuführen, während sich das Ultraschall-Handstück (14) in einem unbelasteten Zustand befindet;

eine zweite Spannung und einen zweiten Strom des Wechselstrom-Antriebssignals, das dem Ultraschall-Handstück (14) zugeführt wird, unter Verwendung des ersten und des zweiten Sensors zu messen, während sich das Ultraschall-Handstück (14) in dem unbelasteten Zustand befindet; und

den Nulllast-Widerstand des Ultraschall-Handstücks (14) auf der Grundlage der gemessenen zweiten Spannung und des gemessenen zweiten Stroms des Wechselstrom-Antriebssignals zu berechnen; oder

ii) Daten, die den Nulllast-Widerstand angeben, aus einem mit dem Ultraschall-Handstück (14) integrierten Speicher (78, 84) auszulesen.

9. Computerprogrammprodukt, das Anweisungen umfasst, die, wenn das Programm von dem Prozessor (52) des Systems nach Anspruch 1 ausgeführt wird, den Prozessor (52) des Systems nach Anspruch 1 dazu veranlassen, die folgenden Schritte auszuführen:

i) Erzeugen und Zuführen (106) eines Wechselstrom-Antriebssignals an ein Ultraschall-Handstück (14) eines Ultraschall-Werkzeugsystems (10), wobei das Wechselstrom-Antriebssignal eingerichtet ist, um Vibrationen einer Spitze (16) des Ultraschall-Handstücks (14) zu verursachen, die nicht ausreichen, um kontaktiertes Gewebe abzutragen;

ii) Empfangen von Rückkopplungsdaten, die dem Wechselstrom-Antriebssignal entsprechen und eine gemessene Spannung des Wechselstrom-Antriebssignals und einen gemessenen Strom des Wechselstrom-Antriebssignals umfassen; und

iii) Bereitstellen von einem akustischen, einem visuellen und/oder einem taktilen Hinweis auf der Grundlage der gemessenen Spannung und des gemessenen Stroms.

10. Computerprogrammprodukt nach Anspruch 9, wobei das Bereitstellen eines akustischen, eines visuellen und/oder eines taktiles Hinweises auf der Grundlage der gemessenen Spannung und des gemessenen Stroms umfasst:

Identifizieren (116) einer Eigenschaft des Patientengewebes auf der Grundlage der gemessenen Spannung und des gemessenen Stroms; und

Bereitstellen (118) eines akustischen, eines visuellen und/oder eines taktilen Hinweises der identifizierten Eigenschaft.

11. Computerprogrammprodukt nach Anspruch 10, wobei das Identifizieren einer Eigenschaft des Patientengewebes auf der Grundlage der gemessenen Spannung und des gemessenen Stroms umfasst:

Berechnen eines Äquivalents des Stroms durch die mechanischen Komponenten des Ultraschallhandstücks (14) auf der Grundlage der gemessenen Spannung und des gemessenen Stroms; und

Identifizieren der Eigenschaft des Patientengewebes auf der Grundlage des berechneten Äquivalents des Stroms durch die mechanischen Komponenten des Ultraschallhandstücks (14); und, optional, wobei das Wechselstrom-Antriebssignal, das dem Ultraschall-Handstück (14) zugeführt wird, eine erste Komponente bei einer Resonanzfrequenz des Ultraschall-Handstücks (14) und eine zweite Komponente bei einer Untersuchungsfrequenz umfasst, die niedriger als die Resonanzfrequenz ist, und das Identifizieren der Eigenschaft des Patientengewebes auf der Grundlage des berechneten Äquivalents des Stroms durch die mechanischen Komponenten des Ultraschall-Handstücks (14) umfasst:

Berechnen einer ersten Amplitude der gemessenen Spannung bei der Untersuchungsfrequenz, einer zweiten Amplitude des berechneten Stromäquivalents durch die mechanischen Komponenten des Ultraschallhandstücks (14) bei der Untersuchungsfrequenz und einer Phasendifferenz zwischen der gemessenen Spannung und dem berechneten Stromäquivalent durch die mechanischen Komponenten des Ultraschallhandstücks (14) bei der Untersuchungsfrequenz; und

Identifizieren der Eigenschaft des Patientengewebes auf der Grundlage der berechneten ersten Amplitude, der berechneten zweiten Amplitude und der berechneten Phasendifferenz.

12. Computerprogrammprodukt nach einem der Ansprüche 9 bis 11, ferner umfassend Anweisungen, die, wenn das Programm durch den Prozessor (52) des Systems nach Anspruch 1 ausgeführt wird, den Prozessor (52) des Systems nach Anspruch 1 dazu veranlassen, einen Schritt auszuführen, der Folgendes umfasst:

Identifizieren der Eigenschaft des Patientengewebes auf der Grundlage der gemessenen Spannung, des gemessenen Stroms und eines Nulllast-Widerstands des Ultraschallhandstücks (14).

13. Computerprogrammprodukt nach einem der Ansprüche 9 bis 12, wobei das Ultraschallwerkzeugsystem (10) ferner einen Speicher (60) umfasst, der Gewebeeigenschaftsdaten (61) speichert, wobei die Gewebeeigenschaftsdaten (61) potenzielle Gewebeeigenschaften und für jede der potenziellen Gewebeeigenschaften einen oder mehrere Werte angeben, die für die potenzielle Gewebeeigenschaft spezifisch sind, und wobei das Bereitstellen eines akustischen, eines visuellen und/oder eines taktilen Hinweises auf Grundlage der gemessenen Spannung und des gemessenen Stroms umfasst:

Identifizieren einer der potentiellen Gewebeeigenschaften, die durch die Gewebeeigenschaftsdaten (61) angegeben werden, als eine Eigenschaft des Patientengewebes, auf der Grundlage des einen oder der mehreren Werte, die für die potenzielle Gewebeeigenschaft spezifisch sind, und der gemessenen Spannung und des gemessenen Stroms; und

Bereitstellen eines akustischen, eines visuellen und/oder eines taktilen Hinweises der identifizierten Eigenschaft des Patientengewebes.

22

**14.** Computerprogrammprodukt nach Anspruch 13, ferner umfassend Anweisungen, die, wenn das Programm durch den Prozessor (52) des Systems nach Anspruch 1 ausgeführt wird, den Prozessor (52) des Systems nach Anspruch 1 dazu veranlassen, einen Schritt auszuführen, der Folgendes umfasst:

Identifizieren der einen der potenziellen Gewebeeigenschaften, die durch die Gewebeeigenschaftsdaten (61) angegeben werden, auf der Grundlage des einen oder der mehreren Werte, die für die potenzielle Gewebeeigenschaft spezifisch sind, der gemessenen Spannung und des gemessenen Stroms und eines Nulllast-Widerstands des Ultraschallhandstücks (14).

**15.** Computerprogrammprodukt nach Anspruch 12 oder 14, ferner umfassend Anweisungen, die, wenn das Programm durch den Prozessor (52) des Systems nach Anspruch 1 ausgeführt wird, den Prozessor (52) des Systems nach Anspruch 1 dazu veranlassen, einen Schritt des Bestimmens des Nulllast-Widerstands des Ultraschall-Handstücks (14) auszuführen, durch:

i) Positionieren des Ultraschallhandstücks (14) in einem unbelasteten Zustand; und während das Ultraschallhandstück (14) in dem unbelasteten Zustand positioniert ist:

Zuführen (106) des Wechselstrom-Antriebssignals zu dem Ultraschall-Handstück (14); Messen einer zweiten Spannung und eines zweiten Stroms des Wechselstrom-Antriebssignals, das dem Ultraschall-Handstück zugeführt wird; und Berechnen des Nulllast-Widerstandes des Ultraschall-Handstücks (14) auf der Grundlage der gemessenen zweiten Spannung und des gemessenen zweiten Stroms des Wechselstrom-Antriebssignals; oder

ii) Auslesen von Daten, die den Nulllast-Widerstand angeben, aus einem mit dem Ultraschall-Handstück (14) integrierten Speicher (78, 84).

**Revendications**

**1.** Système d'outil à ultrasons (10) permettant de sonder le tissu de patient, le système (10) comprenant:

une pièce à main à ultrasons (14) comprenant un embout (16) ayant une région distale (17) pour traiter les tissus de patient et au moins un circuit d'attaque (28) auquel l'embout (16) est couplé et auquel un signal d'attaque CA est appliqué pour faire vibrer l'embout (16), la pièce à main à ultrasons (14) définissant une première voie pour apporter une aspiration à la région distale (17) de l'embout (16) et une seconde voie pour apporter un fluide à la région distale (17) de l'embout (16); et une console de commande (12) couplée à la pièce à main à ultrasons (14) et conçue pour générer le signal d'attaque CA appliqué à l'au moins un circuit d'attaque (28) de la pièce à main à ultrasons (14) pour faire vibrer l'embout (16) de la pièce à main à ultrasons (14), la console de commande (12) comprenant:

un premier capteur pour mesurer la tension du signal d'attaque CA; un second capteur pour mesurer le courant du signal d'attaque CA; et un processeur (52) couplé aux premier et second capteurs et configuré pour:

fournir (106) le signal d'attaque CA à l'au moins un circuit d'attaque (28) de la pièce à main à ultrasons (14), le signal d'attaque CA induisant des vibrations à la région distale (17) de l'embout (16) qui sont insuffisantes pour effectuer l'ablation du tissu du patient; mesurer (108) la tension et le courant du signal d'attaque CA à l'aide des premier et second capteurs; et fournir au moins une indication sonore, visuelle ou tactile en fonction de la tension et du courant mesurés.

**2.** Système (10) selon la revendication 1, dans lequel le processeur (52) est configuré pour fournir au moins une indication sonore, visuelle ou tactile en fonction de la tension et du courant mesurés en étant configuré pour:

identifier une propriété du tissu du patient sur la base de la tension et du courant mesurés; et fournir au moins un élément parmi une indication sonore, visuelle ou tactile de la propriété identifiée.

**3.** Système (10) selon la revendication 2, dans lequel le processeur (52) est configuré pour identifier une propriété du tissu du patient sur la base de la tension et du courant mesurés en étant configuré pour:

calculer un équivalent de courant à travers les composants mécaniques de la pièce à main à ultrasons (14) sur la base de la tension mesurée et du courant mesuré; et

identifier la propriété du tissu du patient sur la base de l'équivalent calculé du courant à travers les composants mécaniques de la pièce à main à ultrasons (14).

4. Système (10) selon la revendication 3, dans lequel le signal d'attaque CA envoyé à la pièce à main à ultrasons (14) comprend une première composante à une fréquence de résonance de la pièce à main à ultrasons (14) et une seconde composante à une fréquence de sondage inférieure à la fréquence de résonance, et le processeur (52) est configuré pour identifier la propriété du tissu du patient sur la base de l'équivalent calculé du courant à travers les composants mécaniques de la pièce à main à ultrasons (14) en étant configuré pour:

calculer une première amplitude de la tension mesurée à la fréquence de sondage, une seconde amplitude de l'équivalent calculé du courant traversant les composants mécaniques de la pièce à main à ultrasons (14) à la fréquence de sondage, et une différence de phase entre la tension mesurée et l'équivalent calculé du courant traversant les composants mécaniques de la pièce à main à ultrasons (14) à la fréquence de sondage; et

identifier la propriété du tissu du patient sur la base de la première amplitude calculée, de la seconde amplitude calculée et de la différence de phase calculée.

5. Système (10) selon l'une quelconque des revendications 1 à 4, dans lequel le processeur (52) est configuré pour fournir au moins une indication sonore, visuelle ou tactile sur la base de la tension et du courant mesurés en étant configuré pour:

identifier une propriété du tissu du patient sur la base de la tension mesurée, du courant mesuré et d'une résistance à vide de la pièce à main à ultrasons (14); et

fournir au moins un élément parmi une indication sonore, visuelle ou tactile de la propriété identifiée.

6. Système (10) selon l'une quelconque des revendications 1 à 5, dans lequel la console de commande (12) comprend en outre une mémoire (60) stockant des données sur les propriétés de tissu (61) couplée au processeur (52), les données sur les propriétés de tissu (61) indiquant les propriétés potentielles de tissu et, pour chacune des propriétés potentielles des tissu, une ou plusieurs valeurs spécifiques à la propriété potentielle de tissu, et le processeur (52) est configuré pour fournir au moins une indication sonore, visuelle ou tactile sur la base de la tension et du courant mesurés en étant configuré pour:

identifier, en tant que propriété du tissu du patient, l'une des propriétés potentielles du tissu indiquées par les données de propriété de tissu (61) sur la base d'une ou plusieurs valeurs spécifiques à la propriété potentielle de tissu et de la tension et du courant mesurés; et

fournir au moins une indication sonore, visuelle ou tactile de la propriété identifiée du tissu du patient.

7. Système (10) selon la revendication 6, dans lequel le processeur (52) est configuré pour identifier l'une des propriétés potentielles de tissu indiquées par les données sur les propriétés de tissu (61) sur la base d'une ou de plusieurs valeurs spécifiques à la propriété potentielle de tissu, de la tension et du courant mesurés et d'une résistance à vide de la pièce à main à ultrasons (14).

8. Système (10) selon la revendication 5 ou 7, dans lequel le processeur (52) est configuré pour déterminer la résistance à vide de la pièce à main à ultrasons (14) en étant configuré pour, en réponse à la pièce à main à ultrasons (14) connectée à la console de commande (12):

i) envoyer le signal d'attaque CA à la pièce à main à ultrasons (14) lorsque la pièce à main à ultrasons (14) est déchargée;

mesurer une seconde tension et un second courant du signal d'attaque CA envoyé à la pièce à main à ultrasons (14) à l'aide des premier et second capteurs lorsque la pièce à main à ultrasons (14) est déchargé; et

calculer la résistance à vide de la pièce à main à ultrasons (14) sur la base de la seconde tension mesurée et du second courant mesuré du signal d'attaque CA; ou

ii) lire les données indiquant la résistance à vide dans une mémoire (78, 84) intégrée à la pièce à main à ultrasons (14).

9. Produit programme informatique comprenant des instructions qui, lorsque le programme est exécuté par le processeur (52) du système selon la revendication 1, amènent le processeur (52) du système selon la revendication 1 à effectuer les étapes suivantes:

i) générer et fournir (106) un signal de commande CA à une pièce à main à ultrasons (14) d'un système d'outils à ultrasons(10), le signal d'attaque CA étant configuré pour provoquer des vibrations d'un embout (16) de la pièce à main à ultrasons (14) qui sont insuffisantes pour effectuer une ablation du tissu en contact;
ii) recevoir des données de retour correspondant au signal d'attaque CA comprenant une tension mesurée du signal d'attaque CA et un courant mesuré du signal d'attaque CA; et
iii) fournir au moins une indication sonore, visuelle ou tactile en fonction de la tension et du courant mesurés.

10. Produit programme informatique selon la revendication 9, dans lequel la fourniture d'au moins une indication sonore, visuelle ou tactile en fonction de la tension et du courant mesurés consiste à:

identifier (116) une propriété du tissu du patient sur la base de la tension et du courant mesurés; et
fournir (118) au moins une indication sonore, visuelle ou tactile de la propriété identifiée.

11. Produit programme informatique selon la revendication 10, dans lequel l'identification du tissu du patient sur la base de la tension et du courant mesurés consiste à:

calculer un équivalent de courant à travers les composants mécaniques de la pièce à main à ultrasons (14) sur la base de la tension mesurée et du courant mesuré; et
identifier la propriété du tissu du patient sur la base de l'équivalent calculé du courant à travers les composants mécaniques de la pièce à main à ultrasons (14); et, éventuellement,
dans lequel le signal d'attaque CA envoyé à la pièce à main à ultrasons (14) comprend une première composante à une fréquence de résonance de la pièce à main à ultrasons (14) et une seconde composante à une fréquence de sondage inférieure à la fréquence de résonance, et l'identification de la propriété du tissu du patient sur la base de l'équivalent calculé du courant à travers les composants mécaniques de la pièce à main à ultrasons (14) consiste à:

calculer une première amplitude de la tension mesurée à la fréquence de sondage, une seconde amplitude de l'équivalent calculé du courant traversant les composants mécaniques de la pièce à main à ultrasons (14) à la fréquence de sondage, et une différence de phase entre la tension mesurée et l'équivalent calculé du courant traversant les composants mécaniques de la pièce à main à ultrasons (14) à la fréquence de sondage; et
identifier la propriété du tissu du patient sur la base de la première amplitude calculée, de la seconde amplitude calculée et de la différence de phase calculée.

12. Produit programme informatique selon l'une quelconque des revendications 9 à 11, comprenant en outre des instructions qui, lorsque le programme est exécuté par le processeur (52) du système selon la revendication 1, amènent le processeur (52) du système selon la revendication 1 à effectuer les étapes suivantes:
identifier la propriété du tissu du patient sur la base de la tension mesurée, du courant mesuré et d'une résistance à vide de la pièce à main à ultrasons (14).

13. Produit programme informatique selon l'une quelconque des revendications 9 à 12, dans lequel le système d'outil à ultrasons (10) comprend en outre une mémoire (60) stockant des données sur les propriétés de tissu (61) les données de propriétés de tissu (61) indiquant des propriétés potentielles de tissu et, pour chacune des propriétés potentielles des tissu, une ou plusieurs valeurs spécifiques à la propriété potentielle de tissu, et la fourniture d'au moins une indication sonore, visuelle ou tactile sur la base de la tension et du courant mesurés consiste à:

identifier, en tant que propriété du tissu du patient, l'une des propriétés potentielles du tissu indiquées par les données de propriété de tissu (61) sur la base d'une ou plusieurs valeurs spécifiques à la propriété potentielle de tissu et de la tension et du courant mesurés; et
fournir au moins une indication sonore, visuelle ou tactile de la propriété identifiée du tissu du patient.

14. Produit programme informatique selon la revendication 13, comprenant en outre des instructions qui, lorsque le programme est exécuté par le processeur (52) du système selon la revendication 1, amènent le processeur (52) du système selon la revendication 1 à effectuer une étape consistant à:

identifier l'une des propriétés potentielles de tissu indiquées par les données sur les propriétés de tissu (61) sur la base d'une ou de plusieurs valeurs spécifiques à la propriété potentielle de tissu, de la tension et du courant mesurés et d'une résistance à vide de la pièce à main à ultrasons (14).

15. Produit programme informatique selon les revendications 12 ou 14, comprenant en outre des instructions qui, lorsque le programme est exécuté par le processeur (52) du système selon la revendication 1, amènent le processeur (52) du système selon la revendication 1 à effectuer une étape de détermination de la résistance à vide de la pièce à main à ultrasons (14):

i) en déchargeant la pièce à main à ultrasons (14); et
tandis que la pièce à main à ultrasons (14) n'est pas chargée:

fournissant (106) le signal d'attaque CA la pièce à main à ultrasons (14);
en mesurant une seconde tension et un second courant du signal d'attaque CA envoyé à la pièce à mains à ultrasons; et
en calculant la résistance à vide de la pièce à main à ultrasons (14) sur la base de la seconde tension mesurée et du second courant mesuré du signal d'attaque CA; ou

ii) en lisant les données indiquant la résistance à vide dans une mémoire (78, 84) intégrée à la pièce à main à ultrasons (14).

# FIG. 1

**FIG. 2A**

**FIG. 2B**

**FIG. 3**

**FIG. 4**

100

Start

102

Mode ──Ablation──→ Operate tip to ablate tissue ─104

Probing

Source probing AC drive signal to ultrasonic instrument ─106

Measure voltage and current of the AC drive signal ─108

Calculate mechanical current ─110

Determine amplitude and phase of modulation voltage and modulation mechanical current ─112

Calculate modulation mechanical resistance ─114

Identify tissue property ─116

Indicate tissue property ─118

# FIG. 5

FIG. 6

**FIG. 7**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20180263652 A1
- US 20120209303 A1
- US 20160361084 A
- US 10016209 B
- US 10449570 B